# EUROPÄISCHE PATENTSCHRIFT

(11) **EP 2 621 505 B1**
(45) Veröffentlichungstag und Bekanntmachung des Hinweises auf die Patenterteilung: **01.06.2016**
(21) Anmeldenummer: 11773680.1
(22) Anmeldetag: 11.10.2011
(51) Int. Cl.: A61K 36/185, A61K 36/28, A61K 36/25, A61K 36/11, A61K 36/53, A61K 36/35, A61K 36/57, A61K 36/235

(54) **VERFAHREN ZUR HERSTELLUNG ALKOHOLFREIER ZUSAMMENSETZUNGEN AUF PFLANZLICHER BASIS SOWIE AUF DIESE WEISE HERGESTELLTE ZUSAMMENSETZUNGEN UND DEREN VERWENDUNG**
METHOD FOR PRODUCING PLANT-BASED ALCOHOL-FREE COMPOSITIONS, COMPOSITIONS PRODUCED IN THIS WAY, AND USE OF SAID COMPOSITIONS
PROCÉDÉ DE FABRICATION DE COMPOSITIONS SANS ALCOOL À BASE DE PLANTES ET COMPOSITIONS FABRIQUÉES DE CETTE MANIÈRE ET LEUR UTILISATION

(30) Priorität: 20.12.2010 DE 102010055170
(43) Veröffentlichungstag der Anmeldung: 07.08.2013
(73) Patentinhaber: Maria Clementine Martin Klosterfrau Vertriebsgesellschaft mbH, 50670 Köln (DE)
(72) Erfinder: NAUERT, Christian, 41542 Dormagen (DE); GREVE, Harald, 40545 Düsseldorf (DE)
(74) Vertreter: Von Rohr Patentanwälte Partnerschaft mbB
(86) Internationale Anmeldenummer: PCT/EP2011/005077
(87) Internationale Veröffentlichungsnummer: WO 2012/084075

(56) Entgegenhaltungen:
- WO-A1-2009/135880
- US-A- 4 933 198
- DATABASE TCM 4. Juli 2005 (2005-07-04), Liu Yuhui et al.: "Antiviral effective part of Radix Isatidis", XP002663455, Database accession no. CN-200510080199-A & CN 1 891 252 A (TIANYIYU PHARMACEUTICAL INVEST [CN]) 10. Januar 2007 (2007-01-10)
- KRAFT K: "Verträglichkeit von Efeublättertrockenextrakt im Kindesalter / Tolerability of dry extracts from ivy (Hedera helix) leaves for children", ZEITSCHRIFT FUER PHYTOTHERAPIE, HIPPOKRATES VERLAG IN MVS MEDIZINVERLAGE, DE, Bd. 25, Nr. 4, 1. Januar 2004 (2004-01-01) , Seiten 179-181, XP009153909, ISSN: 0722-348X
- MENORET Y ET AL: "Nouvelles boissons a teneur reduite en alcool /", COMPTES RENDUS DES SEANCES DE L'ACADEMIE D'AGRICULTURE DEFRANCE, Nr. 5, 1. Januar 1990 (1990-01-01), Seiten 101-110, XP009153895, ACADEMIE D'AGRICULTURE DE FRANCE, XX ISSN: 0001-3986
- BRAND N: "Alcohol in medicines", ZEITSCHRIFT FUER PHYTOTHERAPIE, Bd. 17, Nr. 2, 1. Januar 1996 (1996-01-01) , Seiten 96,99-102,105, XP009153908, HIPPOKRATES VERLAG IN MVS MEDIZINVERLAGE, DE ISSN: 0722-348X
- DATABASE WPI Week 201054, Derwent Publications Ltd., London, GB; AN 2010-J73056 & CN 101 747 995 A (SHENZHEN POLYTECHNIC) 23 Juni 2010
- BASSANI V ET AL: "Preparation of a low-ethanol extract of Atropa belladonna L. using a reverse osmosis membrane", PHARMAZIE 1992 DE, vol. 47, no. 3, 1992, pages 218-220, XP001526572, ISSN: 0031-7144
- DATABASE WPI Week 201013, Derwent Publications Ltd., London, GB; AN 2010-B76410 & JP 2010 029757 A (MIURA KOGYO KK) 12 Februar 2010

## Beschreibung

Die vorliegende Erfindung betrifft das Gebiet der Medizin, insbesondere das Gebiet der prophylaktischen und/oder therapeutischen Behandlung von Atemwegserkrankungen, Erkältungskrankheiten sowie grippalen Infekten und damit verbundenen Symptomen.

Insbesondere betrifft die vorliegende Erfindung ein Verfahren zur Herstellung einer zumindest im Wesentlichen alkoholfreien Zusammensetzung auf Basis mindestens eines ausgewählten Pflanzenextraktes mit sekretolytischer und/oder expektorationsfördernder Wirkung.

Des Weiteren betrifft die vorliegende Erfindung eine zumindest im Wesentlichen alkoholfreie Zusammensetzung auf Basis mindestens eines ausgewählten Pflanzenextraktes mit sekretolytischer und/oder expektorationsfördernder Wirkung, welche sich zur prophylaktischen und/oder therapeutischen Behandlung von Atemwegserkrankungen, Erkältungskrankheiten sowie grippalen Infekten und damit verbundenen Symptomen eignet.

Weiterhin betrifft die vorliegende Erfindung die Verwendung einer zumindest im Wesentlichen alkoholfreien Zusammensetzung auf Basis mindestens eines ausgewählten Pflanzenextraktes mit sekretolytischer und/oder expektorationsfördernder Wirkung zur Herstellung eines Arzneimittels zur Behandlung von Atemwegserkrankungen, Erkältungskrankheiten sowie grippalen Infekten und damit verbundenen Symptomen.

Der Begriff der Atemwegserkrankungen ist im Rahmen der vorliegenden Erfindung sehr weit zu verstehen und bezeichnet insbesondere sämtliche, insbesondere mit der Bildung von zähem Schleim und/oder Schleimhautentzündungen einhergehenden Erkrankungen der oberen wie unteren Atemwege, wobei hierunter insbesondere akute wie chronische entzündliche Krankheitszustände der Atemwege zu subsumieren sind. Beispiele für Atemwegserkrankungen der oberen Atemwege sind z. B. Erkältungen (z. B. Rhinitis) und grippale Infekte, während Beispiele für Atemwegserkrankungen der unteren Atemwege z. B. Bronchitis, Pneumonien oder dergleichen sind.

Der grippale Infekt, welcher im Rahmen der vorliegenden Erfindung synonym auch als Erkältung, Erkältungserkrankung bzw. Erkältungsinfekt bezeichnet wird, stellt insbesondere eine akute Infektionskrankheit insbesondere der Nasen-, Halsund/oder Bronchialschleimhäute dar. Erkältungserkrankungen sind die häufigste Infektion des Menschen überhaupt: So erkranken Kleinkinder mehr als zehnmal im Jahr, während Erwachsene im Schnitt etwa zwei- bis dreimal im Jahr an einem grippalen Infekt erkranken.

Erreger für Erkältungserkrankungen sind im Allgemeinen Viren; zudem kann eine Erkältung zusätzlich auch von Bakterien verursacht bzw. zumindest überlagert werden. Was die als Krankheitsverursacher in Frage kommenden Viren anbelangt, so kommen diesbezüglich sehr verschiedene Viren aus unterschiedlichen Virusfamilien in Betracht, welche sich insbesondere durch eine besonders schnelle Vermehrung bzw. Replikation unter Ausbildung vieler unterschiedlicher Varianten auszeichnen. Beispielsweise können Viren aus der Familie der *Coronaviridae, Andenoviridae, Picornaviridae* und *Paramyxoviridae* Erkältungserkrankungen hervorrufen. Die Vielzahl verschiedener Viren und ihrer Subtypen erklärt, warum Menschen häufig an einer virusbedingten Erkältung erkranken können. Denn eine generelle Ausbildung einer Immunität ist aufgrund der hohen Anzahl und Variabilität der Viren nicht möglich. Die krankheitsverursachenden Viren werden sowohl als Tröpfcheninfektion durch die Luft als auch direkt oder indirekt durch Kontakt mit Erkrankten oder über kontaminierte Gegenstände per Kontaktinfektion oder Schmierinfektion übertragen.

Was den Verlauf der Erkältungserkrankung anbelangt, so kann dieser in zeitliche Phasen untergliedert werden. Die ersten Anzeichen eines grippalen Infektes sind meistens Halskratzen bis hin zu Halsschmerzen und Schluckbeschwerden, oft verbunden mit leichtem Frösteln. Als besonders typisches Erkältungssymptom tritt sehr oft zur gleichen Zeit eine Entzündung der Nasenschleimhäute auf, die auch Schnupfen (Rhinitis) genannt wird. Fast immer treten über einen Zeitraum von vier bis fünf Tagen Kopf- und Gliederschmerzen auf, und die Erkrankten fühlen sich häufig matt und abgeschlagen oder entwickeln sogar Fieber. Im Zuge von Erkältungen bzw. grippalen Infekten entwickelt sich meistens zusätzlich ein Reizhusten. Mit fortschreitender Infektion kommt es zur Bildung von zähflüssigem Schleim, welcher im Laufe der Genesung in Form von Auswurf durch unter Umständen schmerzhaftes Abhusten aus dem Körper entfernt wird.

Auch Atemwegserkrankungen der unteren Atemwege, wie z. B. Bronchitis, Asthma bronchiale, Pneumonien etc., sind häufig mit der Bildung von zähflüssigem Schleim sowie Schleimhautentzündungen und einem unangenehmen Husten verbunden.

Bei der Bronchitis handelt es sich um eine akute oder chronische Entzündung der Bronchien, welche im Krankheitsverlauf mit einer nachfolgenden Erkrankung der Bronchiolen verbunden sein kann. Die akute Bronchitis tritt meist in Verbindung mit Viruserkrankungen, wie z. B. Rhinitis, als so genannte bakterielle Superinfektion auf. Ähnlich wie Atemwegserkrankungen der oberen Atemwege geht auch die Bronchitis im Allgemeinen mit Husten, Auswurf, einer leichten bis mittelschweren Temperaturerhöhung oder sogar Fieber, Thoraxschmerzen, zähem Sputum sowie Rasselgeräuschen beim Atmen einher.

Die bestehenden Therapieansätze und Therapiekonzepte zur Linderung der mit Atemwegserkrankungen einhergehenden Symptome sind im Allgemeinen unzureichend, da sie vielfach zu Unverträglichkeiten führen oder nicht ausreichend effektiv sind.

Die im Allgemeinen eingesetzten medikamentösen Therapien basieren häufig auf synthetischen Wirkstoffen, welche infolge ihrer chemischen Aktivität dauerhaften Schaden im Körper anrichten können. Weiterhin sind synthetische Mittel aufgrund ihrer komplizierten und langen Entwicklung und Herstellungsverfahren häufig deutlich kostenintensiver als naturbasierte Wirkstoffe bzw. Phytopharmaka.

Jedoch gehen auch mit pflanzlichen Wirkstoffen bzw. Phytopharmaka zahlreiche Probleme einher, da insbesondere zur Herstellung flüssiger Dosierungsformen die Wirkstoffe oftmals in Form von alkoholischen Extrakten mit relativ hohem Alkoholgehalt vorliegen. Eine effiziente Extraktion der medizinisch relevanten Inhaltsstoffe ist meist nämlich aufgrund der chemischen Eigenschaften der Wirk- bzw. Inhaltsstoffe nur unter Verwendung alkoholischer Extraktionsmittel möglich. Die Verabreichung alkoholischer Medikamente mit hohem Alkoholgehalt stellt in zahlreichen Fällen jedoch ein gewisses Problem dar. Insbesondere für Säuglinge, Kleinkinder oder Kinder im Schulalter muss infolge des hohen Alkoholgehalts häufig auf synthetische und somit weniger verträgliche Medikationen zurückgegriffen werden. Weiterhin ist aufgrund der Rückfallgefahr die Einnahme alkoholhaltiger Medikamente mit hohem Alkoholgehalt für Alkoholiker nicht möglich. Auch Schwangerschaften oder Stillzeiten stellen Kontraindikationen zur oralen Anwendung bzw. Einnahme von alkoholhaltigen Medikamenten mit hohem Alkoholgehalt dar. Weiterhin kann die Einnahme alkoholischer Medikamente mit hohem Alkoholgehalt das Bewusstsein derart beeinträchtigen, dass das sichere Bedienen von Maschinen oder die Verkehrstauglichkeit nicht mehr gegeben sind.

Zwar existieren inzwischen durchaus Darreichungsformen, welche nur wenig oder sogar gar keinen Ethanol enthalten, aber dies nur aufgrund der Verwendung anderer, ebenfalls alkoholischer Extraktionsmittel, z. B. auf Glykol-Basis, wie Glycerin oder dergleichen.

Somit besteht ein Bedarf an Arzneimitteln oder pharmazeutischen Zusammensetzungen, insbesondere auf pflanzlicher Basis und bevorzugt mit sekretolytischer und/oder expektorationsfördernder Wirkung, welche keinen Ethanol und vorzugsweise auch keine anderen alkoholischen Lösungsmittel aufweisen.

Es hat auch nicht an Versuchen gemangelt, alkoholfreie Medikationen auf Basis von alkoholischen Pflanzenflüssigextrakten herzustellen, indem die flüchtigen zur Gewinnung der medizinisch wertvollen Inhalts- bzw. Wirkstoffe notwendigen Extraktionsmittel, wie beispielsweise Ethanol, durch Vakuumtrocknung, Gefriertrocknung oder ähnliche Verfahren entfernt werden. Zwar können oftmals die alkoholischen Bestandteile erfolgreich entfernt werden, allerdings werden zum Teil auch die medizinisch wirksamen Inhalts- bzw. Wirkstoffe den resultierenden Extrakten entzogen, wodurch diese zu therapeutischen Zwecken nicht immer uneingeschränkt verwendbar sind.

Die CN 1 891 252 A betrifft ein Verfahren zur Herstellung eines antiviralen Mittels aus *Radix isatidis* auf Basis eines Refluxverfahrens mit Ethanol sowie durch Aufkochen des medizinischen Restes mit Wasser, Filtration, Reinigung mit Hilfe eines Harzes *("resin")* als Absorptionsmaterial und Aufkonzentrierung mittels Membranfiltration.

Der wissenschaftliche Artikel gemäß KRAFT, K.: Verträglichkeit von Efeublättertrockenextrakt im Kindesalter, Zeitschrift für Phythotherapie, Bd. 25, Nr. 4, 1. Januar 2004, S. 179-181, ISSN: 0722-348X betrifft die Verträglichkeit von alkoholfreiem Efeublättertrockenextrakt mit sekretolytischer und/oder expektorationsfördernder Wirkung in einer Studie mit Kindern zwischen 0 und 12 Jahren. Bei Trockenextrakten ist die Zusammensetzung der pharmakologisch aktiven Wirkstoffe gegenüber alkoholischen Extrakten verändert.

Die WO 2009/135880 A1 betrifft die Herstellung eines Extraktes von *Cistus creticus* mit mindestens 40 % (m/m) Polyphenolen durch Extraktion mit Alkohol, zumindest teilweises Entfernen des Extraktionsmittels, Rücklösen in wässrigem Lösungsmittel, Filtration und Aufkonzentrierung mittels Membranfiltration.

Der Fachartikel gemäß BRAND, N.: "Alkohol in Arzneimitteln", Zeitschrift für Phytotherapie, Bd. 17, Nr. 2, 1. Januar 1996, S. 96 - 105, ISSN: 0722-348X thematisiert die Risiken von Ethanolersatzstoffen, wie Polyethylenglykol oder Glycerol, in Bezug auf pflanzliche Tinkturen und Liquida. In diesem Zusammenhang wird eine Warnung dahingehend ausgesprochen, die erprobten ethanolhaltigen Präparate durch unerprobte alkoholfreie Zubereitungen, hergestellt mit Ethanolersatzstoffen, zu ersetzen.

Weiterhin betrifft die CN 101747995 Thymianextrakt, welcher aus Thymian-Keimlingen bzw. ausgekeimten Thymian-Samen mittels organischer Lösemittel extrahiert wird. Das organische Lösemittel wird durch Verdampfen aus der Extraktionsmischung entfernt. Anschließend wird die Extraktmischung zum Erhalt der Thymianessenz durch eine organische Filtermembran filtriert.

Die wissenschaftliche Publikation gemäß Bassani et al.: *"Preparation of* a *lowethanol extract of Atropa belladonna L. using a reverse osmosis membrane"* betrifft einen alkaloidhaltigen Extrakt aus *Atropa belladonna,* dessen Ethanolkonzentration mittels Querstromfiltration bzw. Cross-Flow-Filtration reduziert wird.

In der JP 2010029757 wird ein Membranfiltrationssystem beschrieben, welches als Apparat zur Durchführung einer Umkehrosmose eingesetzt werden kann. Dabei kann die Zufuhr eines nicht näher spezifizierten Dispergiermittels vorgesehen sein.

Auch andere Verfahren zur Entfernung der Auszugsmittel bzw. des Alkohols aus pflanzlichen Extrakten (wie z. B. destillative Verfahren, Evaporationsverfahren, Lyophilisationsverfahren etc.) konnten sich bislang aufgrund der mangelnden Effizienz sowie der Unwirtschaftlichkeit dieser Verfahren nicht durchsetzen.

Somit besteht immer noch ein großer Bedarf an gut verträglichen Arzneimitteln auf der Basis von pflanzlichen Extrakten, insbesondere auf pflanzlicher Basis und bevorzugt mit sekretolytischer und/oder expektorationsfördernder Wirkung, welche jedoch keinen Ethanol und vorzugsweise auch keine sonstigen alkoholischen Lösungsmittel bzw. Extraktionsmittel aufweisen.

Daher liegt der vorliegenden Erfindung die Aufgabe zugrunde, ein Verfahren zur Herstellung eines Arzneimittels und ein nach diesem Verfahren erhältliches Arzneimittel als solches bereitzustellen, wobei sich das Arzneimittel zur Behandlung von Atemwegserkrankungen, Erkältungskrankheiten sowie grippalen Infekten und damit verbundenen Symptomen eignen soll und einerseits pflanzliche Inhaltsstoffe bzw. Phytopharmaka in medizinisch wirkungsvoller Menge enthält, aber andererseits einen derart geringen Alkohol- bzw. Ethanolgehalt bzw. zumindest im Wesentlichen keinen Alkohol, insbesondere keinen Ethanol, aufweist, dass die zuvor geschilderten, im Zusammenhang mit dem Stand der Technik auftretenden Nachteile und Nebenwirkungen wenigstens teilweise vermieden oder aber wenigstens zumindest teilweise abgeschwächt werden sollen.

Zur Lösung des zuvor geschilderten Problems schlägt die vorliegende Erfindunggemäß einem **ersten** Erfindungsaspekt - ein Verfahren zur Herstellung einer zumindest im Wesentlichen alkoholfreien Zusammensetzung auf Basis mindestens eines Pflanzenextraktes mit sekretolytischer und/oder expektorationsfördernder Wirkung gemäß Anspruch 1 vor; weitere vorteilhafte Ausgestaltungen dieses Erfindungsaspekts sind Gegenstand der diesbezüglichen Unteransprüche.

Weiterer Gegenstand der vorliegenden Erfindung - gemäß einem **zweiten** Erfindungsaspekt - ist eine zumindest im Wesentlichen alkoholfreie Zusammensetzung auf Basis mindestens eines Pflanzenextraktes mit sekretolytischer und/oder expektorationsfördernder Wirkung gemäß Anspruch 9 bzw. 10, welche sich insbesondere zur prophylaktischen und/oder therapeutischen Behandlung von Atemwegserkrankungen, Erkältungskrankheiten sowie grippalen Infekten und damit verbundenen Symptomen eignet; weitere vorteilhafte Ausgestaltungen dieses Erfindungsaspekts sind Gegenstand der diesbezüglichen Unteransprüche.

Wiederum weiterer Gegenstand der vorliegenden Erfindung - gemäß einem **dritten** Erfindungsaspekt - ist die Verwendung einer erfindungsgemäßen Zusammensetzung zur Herstellung eines Arzneimittels zur prophylaktischen und/oder therapeutischen Behandlung von Atemwegserkrankungen, Erkältungskrankheiten sowie grippalen Infekten und damit verbundenen Symptomen gemäß Anspruch 15.

Es versteht sich von selbst, dass besondere Ausgestaltungen und Ausführungsformen, welche nur im Zusammenhang mit einem Erfindungsaspekt beschrieben sind, auch in Bezug auf die anderen Erfindungsaspekte entsprechend gelten, ohne dass dies ausdrücklich beschrieben ist.

Bei allen nachstehend genannten relativen bzw. prozentualen, insbesondere gewichtsbezogenen Mengenangaben ist zu beachten, dass diese Angaben im Rahmen der erfindungsgemäßen Zusammensetzung vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Im Übrigen gilt, dass der Fachmann anwendungsbezogen oder einzelfallbedingt von den nachfolgend ausgeführten Mengenangaben abweichen kann, ohne dass er den Rahmen der vorliegenden Erfindung verlässt.

Gegenstand der vorliegenden Erfindung - gemäß einem **ersten** Aspekt der vorliegenden Erfindung - ist somit ein Verfahren zur Herstellung einer zumindest im Wesentlichen alkoholfreien Zusammensetzung auf Basis mindestens eines Pflanzenextraktes mit sekretolytischer und/oder expektorationsfördernder Wirkung, wobei die Zusammensetzung einen Alkoholgehalt, insbesondere einen Ethanolgehalt, von weniger als 2 Vol.-% aufweist,
wobei das Verfahren die folgenden Verfahrensschritte umfasst:
(a) Bereitstellung mindestens eines alkoholischen, insbesondere ethanolischen Pflanzenextraktes mit sekretolytischer und/oder expektorationsfördernder Wirkung mit einem Droge/Extrakt-Verhältnis im Bereich von 1 : 10 bis 10 : 1, wobei der mindestens eine Pflanzenextrakt mit sekretolytischer und/oder expektorationsfördernder Wirkung ausgewählt ist aus der Gruppe von Thymian-Extrakt *(Thymus vulgaris),* Primelwurzel-Extrakt *(Primula),* Efeu-Extrakt (*Hedera helix),* Holunder-Extrakt (*Sambucus nigra*), Fenchel-Extrakt (*Foeniculum vulgare*), Huflattich-Extrakt (*Tussilago farfara*), Anis-Extrakt (*Pimpinella anisum*) und Tüpfelfarn-Extrakt (*Polypodium vulgare*) sowie Mischungen und Kombinationen von mindestens zwei der vorgenannten Extrakte; und
(b) nachfolgend Entfernen des Alkohols, insbesondere des Ethanols, mittels Membranfiltration zum Erhalt einer im Wesentlichen alkoholfreien Zusammensetzung mit einem Alkoholgehalt, insbesondere einem Ethanolgehalt, von weniger als 2 Vol.-%, auf Basis mindestens eines Pflanzenextraktes mit sekretolytischer und/oder expektorationsfördernder Wirkung, wobei vor der Durchführung der Membranfiltration mindestens ein Dispergier- oder Netzmittel, vorzugsweise ein Lösungsvermittler, zugesetzt wird und/oder wobei die Membranfiltration in Gegenwart mindestens eines Dispergier- oder Netzmittels, vorzugsweise Lösungsvermittlers, durchgeführt wird, wobei das Dispergier- oder Netzmittel ausgewählt ist aus der Gruppe von organischen Ölen, Fetten und Fettsäuren, Lecithinen, Glyceriden sowie deren Kombinationen und wobei das Dispergier- oder Netzmittel in Mengen von 0,1 bis 30 Gew.-% eingesetzt wird, bezogen auf die der Membranfiltration zu unterziehende Gesamtmischung aus alkoholischem Pflanzenextrakt, Additiv, Wasser und gegebenenfalls weiteren Inhaltsstoffen.

Überraschenderweise wurde im Rahmen der vorliegenden Erfindung von der Anmelderin gefunden, dass sich durch das erfindungsgemäße Verfahren, welches unter anderem den Verfahrensschritt des vorzugsweise selektiven Entfernens des Alkohols, insbesondere des Ethanols, mittels Membranfiltration aus mindestens einem alkoholischen, insbesondere ethanolischen Pflanzenextrakt umfasst, eine Zusammensetzung auf Basis mindestens eines Pflanzenextraktes mit sekretolytischer und/oder expektorationsfördernder Wirkung herstellen lässt, welche zumindest im Wesentlichen alkoholfrei ist und zur Behandlung von Atemwegserkrankungen, Erkältungskrankheiten, grippalen Infekten und den damit verbundenen Symptomen eingesetzt werden kann.

Dies ist insbesondere vollkommen überraschend, da im Fall von alkoholischen, insbesondere ethanolischen Pflanzenextrakten mit sekretolytischer und/oder expektorationsfördernder Wirkung nach dem Stand der Technik eine Entfernung des Alkohols oder des alkoholischen Extraktionsmittels nicht bzw. nicht ohne Weiteres (insbesondere nicht unter Substanzverlust bzw. Verlust der Extrakt-Inhaltsstoffe) möglich ist, weil die in diesen Extrakten enthaltenen Wirk- und Inhaltsstoffe mit sekretolytischer und/oder expektorationsfördernder Wirkung (z. B. aromatische Verbindungen, ätherische Öle etc.) im Allgemeinen volatil sind und bei der Entfernung des Alkohols oder des alkoholischen Extraktionsmittels mitgerissen würden. Im Rahmen der vorliegenden Erfindung ist es aber unerwarteterweise gelungen, dieses Problem in effizienter Weise zu lösen.

Der Begriff "pharmazeutische Zusammensetzung" - synonym bisweilen auch als "pharmazeutische Zubereitung" bezeichnet - ist im Rahmen der vorliegenden Erfindung sehr weit zu verstehen und bezeichnet nicht nur Arzneimittel, Medikamente, Pharmaka oder sonstige Medizinprodukte, welche laut Arzneimittelgesetz zur Diagnostik bzw. als Therapeutika bestimmt sind, sondern darüber hinaus auch Nahrungsergänzungsmittel oder sonstige nicht unter das Arzneimittelgesetz fallende Produkte, Zubereitungen oder Zusammensetzungen.

Der Begriff "zumindest im Wesentlichen alkoholfrei" ist diesbezüglich dahingehend zu verstehen, dass gemäß der deutschen Arzneimittelwarenverordnung der Ethanolgehalt der pharmazeutischen Zusammensetzung weniger als 0,05 g pro maximaler Einzeldosis beträgt. Weiterhin berücksichtigt der Begriff "zumindest im Wesentlichen alkoholfrei" auch die gemäß dem Europäischen Arzneibuch vorgegebene maximale Konzentration von verbleibenden Lösungsmitteln der Klasse 3, welche Ethanol umfassen, wonach eine maximale Konzentration des Ethanols von 5 g/kg bzw. 5.000 ppm im finalen Produkt gestattet ist, ohne dass die pharmazeutische Zusammensetzung als alkoholhaltig deklariert werden muss. Die Einnahme von Arzneimitteln mit Alkohol- bzw. Ethanolgehalten dieser Größenordnung ist auch bei Vorliegen der genannten Kontraindikationen unbedenklich.

Der Begriff "zumindest im Wesentlichen alkoholfrei" bezieht sich insbesondere aber nicht nur auf den Ethanolgehalt, sondern vorzugsweise vielmehr auf sämtliche alkoholische Substanzen bzw. Extraktionsmittel, welche insbesondere dem Auszug der pflanzlichen Wirk- und Inhaltsstoffe dienen können. Somit sind auch organische Lösungsmittel auf Glykol-Basis, wie z. B. Glycerin oder Polyethylenglykol, mitumfasst.

Der Begriff des selektiven Entfernens des Alkohols bzw. Ethanols ist im Rahmen der vorliegenden Erfindung insbesondere dahingehend zu verstehen, dass zumindest im Wesentlichen ausschließlich der Alkohol bzw. Ethanol entfernt wird, insbesondere die übrigen Inhaltsstoffe der Pflanzenextrakte zu mindestens 90 Gew.-%, insbesondere zu mindestens 95 Gew.-%, vorzugsweise zu mindestens 96 Gew.-%, besonders bevorzugt zu mindestens 98 Gew.-% oder sogar mehr, erhalten bleiben bzw. nicht entfernt werden.

Was die Begriffe der "Sekretolyse" und der "Expektoration" anbelangt, so ist diesbezüglich Folgendes auszuführen:
Die vorgenannten, im Rahmen der vorliegenden Erfindung zu therapierenden Atemwegserkrankungen gehen aufgrund der Bildung von Sekreten mit einer mehr oder weniger stark ausgeprägten Expektoration von Sputum oder Bronchialsekret einher, welcher bzw. welches insbesondere Leukozyten, Epithelzellen, Staubteilchen, Rauchpartikel, Mikroorganismen etc. enthält. Der Begriff der Expektoration bezeichnet dabei das Aushusten dieser Sekrete bzw. des Sputums aus dem Bronchialsystem; die Expektoration spielt für den Heilungsprozess eine zentrale Rolle, da auf diesem Wege ein Großteil der Krankheitserreger, wie z. B. Viren oder Bakterien, aus dem Körper befördert wird. Um einen erleichterten Auswurf des Sputums zu ermöglichen, kann der Prozess der Sekretolyse, welcher die Auflösung des zähen Schleims bezeichnet, durch die Gabe von Expektorantien (d. h. auswurffördernden Mitteln) oder sekretolytischen Mitteln bzw. Sekretolytika (d. h. schleimlösenden Mitteln) unterstützt und somit beschleunigt werden. Als Expektorantien werden im Rahmen der vorliegenden Erfindung insbesondere solche auswurffördernden Mittel bezeichnet, welche entweder einen sekretolytischen (d. h. also die Verflüssigung des Bronchialsekrets bewirkenden) Effekt oder aber einen sekretomotorischen (d. h. einen verstärkten Abtransport des Bronchialschleims bewirkenden) Effekt aufweisen.

Für weitere diesbezügliche Informationen zu den Begriffen der "Sekretolyse" und der "Expektoration" kann insbesondere verwiesen werden auf Pschyrembel, Klinisches Wörterbuch, Nikol Verlagsgesellschaft mbH, Hamburg, 257. Auflage, Stichwörter: *"Expektoration", "Expektorantien", "Sputum"* und *"Sekretolytika",* sowie die dort jeweils referierte Literatur, deren jeweiliger Inhalt hiermit durch Bezugnahme eingeschlossen ist.

Da eine beschleunigte Expektoration und eine geförderte Sekretolyse die Krankheitsdauer maßgeblich verkürzen, zielen zahlreiche Therapieansätze zur Behandlung von Atemwegserkrankungen insbesondere auf eine Unterstützung der Sekretolyse bzw. Unterstützung der Expektoration ab. Dies beinhaltet nach dem Stand der Technik insbesondere die Applikation synthetischer Wirkstoffe zur Verflüssigung des Bronchialsekrets. Weiterhin existieren auch phytopharmazeutische Ansätze zur Beschleunigung der Expektoration auf Basis von Wirkstoffen aus Heilpflanzen bzw. entsprechenden Arzneidrogen, welche meist weitaus verträglicher als synthetische Wirkstoffe sind.

Im Rahmen der vorliegenden Erfindung wird der mindestens eine Pflanzenextrakt mit sekretolytischer und/oder expektorationsfördernder Wirkung aus der Gruppe von Thymian-Extrakt, Primelwurzel-Extrakt, Efeu-Extrakt, Holunder-Extrakt, Fenchel-Extrakt, Huflattich-Extrakt, Anis-Extrakt und Tüpfelfarn-Extrakt sowie Mischungen und Kombinationen von mindestens zwei der vorgenannten Extrakte ausgewählt. Erfindungsgemäß bevorzugt sind Thymian-Extrakt und/oder Primelwurzel-Extrakt, insbesondere eine Kombination oder Mischung von Thymian-Extrakt und Primelwurzel-Extrakt.

Was die erfindungsgemäß eingesetzten Extrakte mit sekretolytischer und/oder expektorationsfördernder Wirkung anbelangt, so ist diesbezüglich Folgendes auszuführen:
Beim Echten Thymian *(Thymus vulgaris)* handelt es sich um eine Pflanzenart aus der Gattung der Thymiane (*Thymus*) aus der Familie der Lippenblütler (*Lami*aceae). Hinsichtlich der chemischen Zusammensetzung seiner ätherischen Öle bzw. der Extrakte wird der Echte Thymian in sechs Chemotypen eingeteilt. Abhängig von klimatischen und genetischen Bedingungen der Pflanzenart sind Thymol, Geraniol, Linalool, α-Terpineol, Trans-thuyanol-4-terpineol-4 oder Carvacrol Hauptbestandteile der ätherischen Öle bzw. der Thymian-Extrakte. Insbesondere der Thymian-Inhaltstoff Thymol zählt zu den stärksten antibakteriell und antiviral wirkenden Einzelkomponenten von ätherischen Ölen und wird daher zur Behandlung von Entzündungen der Mund- und Rachenschleimhäute, Mundgeruch sowie kleineren Wunden eingesetzt. Weiterhin besitzt Thymol krampflösende, sekretionsfördernde und schmerzlindernde Eigenschaften, was insbesondere hinsichtlich des Einsatzes zur Behandlung von Katarrhen der oberen Luftwege, Erkältungskrankheiten bzw. grippalen Infekten, Bronchitis und Keuchhusten von Relevanz ist. Zudem werden den Polymethoxyflavonen und Monoterpenen des Thymians ebenfalls eine krampflösende und hustenreizstillende Wirkung zugesprochen.

Primeln (*Primula*) wiederum sind eine Pflanzengattung aus der Familie der Primelgewächse (*Primulaceae*), welche aufgrund ihres hohen Saponingehalts, insbesondere ihres hohen Primulasäuregehalts, auch als Heilpflanzen verwendet werden. Weiterhin enthalten Primeln wertvolle Inhaltsstoffe des pflanzlichen Sekundärmetabolismus, wie etwa Flavonoide und Carotinoide, sowie Spuren von ätherischem Öl und Enzymen wie der Primverase. Tee auf Basis von Bestandteilen der Primeln wirkt als mildes Sekretolytikum bei Husten, Bronchitis oder Erkältungskrankheiten. Primelwurzeln werden besonders häufig aufgrund ihres hohen Saponingehalts zur Gewinnung von Extrakten eingesetzt. Die Extrakte werden schließlich als Phytotherapeutika zur Verflüssigung des Sputums bei Erkrankungen der Atemwege mit Hustenreiz in Form von Hustentee, -säften und -tropfen verwendet.

Beim Efeu - systematisch *Hedera helix -* handelt es sich um eine immer grüne Mauerranke, welche in ganz Europa sowie Westasien vorkommt und deren Blätter häufig zur Herstellung von Phytopharmaka Verwendung finden. Die Blätter enthalten 5 bis 8 Gew.-% bidesmosidische Saponine, insbesondere Oleanolsäure, Hederagenin und Bayogenin, als pharmazeutisch wertvolle Inhaltsstoffe. In trockenen Blättern können sich durch Hydrolyse zudem die stärker wirkenden monodesmosidischen Saponine bilden. Weiterhin liegen im Efeu verschiedene Phenolverbindungen, Polyacetylen, Falcarinol, Falcarinon und 11,12-Dehydrofalcarinol vor. Die stark expektorierenden Eigenschaften der Saponine des Efeus werden durch eine indirekte Reizung des *Nervus vagus* im Magen erklärt. Die Anwendung erfolgt meist unter Verwendung der Blattextrakte, insbesondere bei Husten, spastischer Bronchitis und chronischem Atemwegskatarrh.

Auch der Holunder - systematisch *Sambucus nigra -* besitzt Wirkstoffe, welche die Expektoration fördern bzw. beschleunigen. Zu diesen Inhaltsstoffen gehören Glycoside, ätherisches Öl, Flavonoide, Cholin, Schleimstoffe, Gerbstoffe, Gerbsäure sowie Vitamin C. Zur Herstellung von Phytopharmaka mit Inhaltsstoffen des Holunders werden insbesondere die Blüten der Pflanze verwendet. Solche Phytopharmaka werden insbesondere bei akuten Krankheiten der Atemwege, wie Husten, Bronchitis, Erkältungskrankheiten, Nebenhöhlenentzündungen oder auch allgemein zur Stärkung des Immunsystems, verwendet, da sie nicht nur schleimlösend, sondern auch entzündungshemmend, pilztötend sowie allgemein anregend wirken.

Eine weitere Pflanze, welche sekretolytische Inhaltsstoffe enthält, ist der Fenchel-systematisch *Foeniculum vulgare.* Hier werden zur Herstellung von Phytopharmaka meistens die Früchte und Wurzeln eingesetzt. Als Inhaltsstoffe enthält Fenchel insbesondere ätherische Öle, aber auch Kieselsäure, Mineralsalze, Stärke sowie die Vitamine A, B und C. Fenchel hat ebenfalls eine stark schleimlösende und auswurffördernde Wirkung auf die Atemwege und hemmt weiterhin das bakterielle Keimwachstum.

Auch der Huflattich - systematisch *Tussilago farfara -* ist für seine pharmakologische Wirksamkeit bekannt. Als wirksame Inhaltsstoffe enthält er unter anderem Polysaccharide, Schleimstoffe, Styrole, Bitterstoffe und Gerbstoffe. Wie die bereits vorgenannten Heilpflanzen wirken Huflattich bzw. dessen Inhaltsstoffe bei Hustenreiz beruhigend und insbesondere schleimlösend. Als arzneilich wirksamer Teil zur Herstellung von Phytopharmaka werden im Allgemeinen die Blätter verwendet.

Beim Anis - *Pimpinella anisum -* handelt es sich um eine Pflanze, welche ursprünglich aus Asien und den südöstlichen Mittelmeerländern stammt und ebenfalls zur Behandlung von Atemwegserkrankungen dient. Vor allem wegen der geschmacklichen Eigenschaften ist Anis zur Herstellung von oral applizierbaren Zusammensetzungen beliebt. Die pharmazeutisch wirksamen Inhaltsstoffe von Anis umfassen vor allem ätherisches Öl, Anethol, Isoanethol, Anisketon, Anissäure, Acetylcholin, Kampfer, Eugenol, Kaffeesäure, Myristicin, Salicylate, Thymol, Xanthotoxin und Vitamin C. Anis ist zudem häufig Bestandteil von Teemischungen, wird aber auch in Form von ätherischem Öl eingesetzt. Die Besonderheit und spezielle Wirksamkeit des ätherischen Öls von Anis liegt insbesondere darin, dass es nach der Einnahme über die Lunge ausgeschieden wird und somit durch die Lungenbläschen und Bronchien von Innen wirkt.

Der Tüpfelfarn - systematisch *Polypodium vulgare -* ist eine Heilpflanze, deren pharmakologische Wirkung bereits seit dem Mittelalter bekannt ist. Heute wird der Tüpfelfarn in erster Linie als Expektorans eingesetzt, da er sich insbesondere zur Behandlung von Bronchitis eignet. Als wirksame Inhaltsstoffe enthält die Pflanze in erster Linie Gerbstoffe, Weichharz, Schleim, Eiweiß, apfelsaures Calcium sowie etwa 8 Gew.-% fettes Öl sowie Glycyrrhizin. Zudem wird den Wirkstoffen des Tüpfelfarns eine partielle hämolytische Wirkung zugeschrieben.

Die vorliegende Erfindung ist mit zahlreichen Vorteilen, Verbesserungen und Besonderheiten verbunden, welche die vorliegende Erfindung gegenüber dem Stand der Technik auszeichnen und welche in nicht beschränkender Weise wie folgt zusammengefasst werden können:
Die vorliegende Erfindung stellt erstmals ein effizientes Verfahren zur Herstellung einer zumindest im Wesentlichen alkoholfreien Zusammensetzung auf Basis mindestens eines Pflanzenextraktes mit sekretolytischer und/oder expektorationsfördernder Wirkung zur Verfügung, wobei sich die resultierende Zusammensetzung insbesondere zur Behandlung von Atemwegserkrankungen, insbesondere Erkältungskrankheiten, grippalen Infekten oder Bronchialerkrankungen etc., eignet. Eine effiziente, ökonomische und selektive Entfernung von Extraktionsmitteln, insbesondere Alkohol, zur Herstellung von alkoholfreien flüssigen Zusammensetzungen auf Basis von pflanzlichen Extrakten war bislang nämlich nicht in dieser Weise möglich.

In besonderem Maße ist dabei hervorzuheben, dass die erfindungsgemäße Zusammensetzung aufgrund des erfindungsgemäßen Verfahrens nicht nur frei von Ethanol ist, sondern vorzugsweise auch keine bzw. zumindest im Wesentlichen keine anderen alkoholischen Lösungs- und Extraktionsmittel, insbesondere keine auf Glykol-Basis, aufweist.

In besonderem Maße ist dabei hervorzuheben, dass im Rahmen des erfindungsgemäßen Verfahrens der Alkohol, insbesondere Ethanol, derart selektiv entfernt wird, dass kein bzw. zumindest im Wesentlichen kein Substanzverlust der pharmazeutisch relevanten Inhaltsstoffe in den pflanzlichen Extrakten auftritt, obwohl diese teilweise nur geringfügig höhere Molekulargewichte als Ethanol besitzen. Dies ist insbesondere überraschend, da eigentlich die Auftrennung von Molekülen mit ähnlichen Molekulargewichten mittels Filtrationsverfahren eine technische Hürde darstellt.

Auch hat es sich als besonders vorteilhaft erwiesen, den Verfahrensschritt der Membranfiltration in einem geschlossenen System, insbesondere in einem geschlossenen Reaktorsystem, durchzuführen. Bei Einsatz von offenen Systemen zur Entfernung von Alkohol aus pflanzlichen Extrakten besteht das Problem, dass es zu etwaigen Verlusten der Wirk- und Inhaltsstoffe kommen kann, da es sich hierbei häufig um volatile Substanzen, wie beispielsweise ätherische Öle, Thymol etc., handelt, die über das offene System aus dem Extrakt evaporieren können. Basierend auf den von der Anmelderin durchgeführten Versuchs- und Forschungsarbeiten konnte dieses Problem im Rahmen der vorliegenden Erfindung jedoch eindrucksvoll überwunden werden.

Weiterhin bedingt die erfindungsgemäße Verfahrensführung, dass durch den Einsatz von Dispergier- oder Netzmitteln, insbesondere Lösungsvermittlern, die Entfernung des Alkohols durch Membranfiltrationsverfahren zeitlich optimiert erfolgen kann, da Dispergier- oder Netzmittel, insbesondere Lösungsvermittler, auch die Membranfiltration von Mischextakten auf Basis von mindestens zwei voneinander verschiedenen Pflanzenextrakten erlauben. Dies ist mit einer deutlich gesteigerten Produktionseffizienz gegenüber der separaten Membranfiltration von Einzelextrakten verbunden. Die Filtration von Mischextrakten war allerdings bislang nicht möglich, da für eine effiziente Herstellung der Extrakte abhängig von den Inhalts- und Wirkstoffen der jeweiligen Pflanze bzw. Arzneidroge vollkommen unterschiedliche Extraktionsmittel verwendet werden müssen, so dass es beim Mischen verschiedener Extrakte zur Präzipitation der Inhaltsstoffe kommen kann. Dies kann jedoch durch das erfindungsgemäß optional verwendete Dispergier- oder Netzmittel, insbesondere den Lösungsvermittler, verhindert werden.

Darüber hinaus bietet das erfindungsgemäße Verfahren den Vorteil, dass die Extraktion des Ethanols bzw. die Herstellung der erfindungsgemäßen Zusammensetzung unter pharmakologisch kontrollierbaren Bedingungen erfolgt.

Die Stofftrennung mittels Membranverfahren geschieht im Allgemeinen bei Umgebungstemperaturen sowie ohne Einsatz von Zusatzstoffen, so dass die Gemische weder thermisch noch chemisch belastet werden. Die Wirk- und Inhaltsstoffe der erfindungsgemäß eingesetzten Pflanzenextrakte bleiben somit in chemischer Hinsicht erhalten, was insbesondere im Hinblick auf die pharmazeutische Wirksamkeit von essentieller Bedeutung ist. Auch ist die Membranfiltration ein vergleichsweise umweltfreundliches und energiesparendes Verfahren.

Somit wird erstmals eine insbesondere pharmazeutische Zusammensetzung auf Basis mindestens eines Pflanzenextraktes mit sekretolytischer und/oder expektorationsfördernder Wirkung bereitgestellt, welche einen derart geringen Alkoholgehalt aufweist bzw. zumindest im Wesentlichen keinen Alkohol enthält, dass auch Patienten mit Kontraindikationen in Bezug auf die Einnahme alkoholhaltiger Medikamente gefahrlos behandelt werden können und auf die Einnahme synthetischer und deutlich stärkere Nebenwirkungen aufweisender Arzneimittel verzichtet werden kann.

Zudem zeichnen sich die pflanzlichen Wirk- bzw. Inhaltsstoffe der vorgenannten Pflanzen bzw. Arzneidrogen, wie beispielsweise das Thymol im Fall des Thymians oder die Saponine im Fall von Primelwurzeln, durch ihre gute Verträglichkeit sowie ihre Effizienz beim Einsatz zur Behandlung von mit Atemwegserkrankungen einhergehenden Symptomen aus, insbesondere bei der Förderung der Expektoration bzw. bei der Beschleunigung der Sekretolyse von mit Atemwegserkrankungen einhergehendem zähem Schleim bzw. Bronchialsekret. Signifikante Nebenwirkungen sind in Bezug auf die Inhaltsstoffe der vorgenannten Pflanzen bzw. deren Extrakten als solche nicht bekannt. Vielmehr ist bei den diesbezüglichen Extrakten des Standes der Technik der hohe Alkohol-Anteil bzw. der hohe Anteil alkoholischer Extraktionsmittel in flüssigen Dosierungsformen das größere Problem, welches aber im Rahmen der Erfindung erfolgreich überwunden wurde.

Auch ist die Gefahr von weiteren Nebenwirkungen in der erfindungsgemäßen Zusammensetzung weiter reduziert, weil sämtliche alkoholische Extraktionsmittel entfernt werden können. In der Folge besteht die Zusammensetzung im Wesentlichen aus einer Kombination lediglich der Wirk- bzw. Inhaltsstoffe der Extrakte sowie Wasser und gegebenenfalls weiteren alkoholfreien Inhaltsstoffen (z. B. Zucker, Zuckeraustauschstoffen etc.).

Weiterhin werden die pflanzlichen Inhalts- und Wirkstoffe der vorgenannten Pflanzen bzw. Arzneidrogen, so zum Beispiel das Thymol des Thymians oder die Saponine der Primelwurzeln, durch das erfindungsgemäße Verfahren in ihren Konzentrationen zumindest im Wesentlichen nicht verändert und liegen in medizinisch erforderlicher Menge in der finalen Zusammensetzung vor, was bislang das zentrale Problem bei der Herstellung alkoholfreier Zusammensetzungen auf Basis dieser pflanzlichen Extrakte darstellt. Die Wirkstoffe bleiben jedoch nicht nur in ihrer Konzentration erhalten, sondern werden auch in chemischer Hinsicht nicht verändert.

Somit wird eine zumindest im Wesentlichen alkoholfreie Zusammensetzung auf Basis mindestens eines Pflanzenextraktes mit sekretolytischer und/oder expektorationsfördernder Wirkung zur Behandlung von Atemwegserkrankungen bereitgestellt, welche nicht nur im Falle des Vorliegens von Kontraindikationen hinsichtlich der Einnahme alkoholhaltiger Medikationen besser geeignet ist, sondern auch bei Patienten ohne Kontraindikationen eine mit alkoholhaltigen Zubereitungen vergleichbare Dosierung bzw. Gabe der Wirk- und Inhaltsstoffe sicherstellt.

Auch die Produktion der erfindungsgemäßen Zusammensetzung ist äußerst ökonomisch, da die Prozessführung äußerst gut zu handhaben ist und die eingesetzten Stoffe, insbesondere die pflanzlichen Extrakte, sehr kostengünstig gewonnen werden können.

Weiterhin zeichnet sich die Zusammensetzung nach Art der vorliegenden Erfindung durch ihre hervorragenden organoleptischen Eigenschaften aus, wie auch die Anwendungsbeobachtungen zeigen. So ist die Zusammensetzung hinsichtlich ihres Geschmacks, aber auch in Bezug auf ihre Konsistenz als äußerst angenehm und darüber hinaus gut handhabbar (z. B. bei der Produktion und Abfüllung sowie bei der Applikation) einzustufen. Dies ist insbesondere dahingehend relevant, dass die Zusammensetzung gerade für Säuglinge und Kinder vorgesehen ist, die häufig die orale Aufnahme von Zubereitungen mit unangenehmem Geschmack verweigern.

Was das erfindungsgemäße Verfahren zur Herstellung einer zumindest im Wesentlichen alkoholfreien Zusammensetzung, insbesondere einer pharmazeutischen Zusammensetzung bzw. pharmazeutischen Zubereitung, auf Basis mindestens eines Pflanzenextraktes mit sekretolytischer und/oder expektorationsfördernder Wirkung anbelangt, so gliedert sich dieses Verfahren - wie zuvor beschrieben - in mindestens zwei Verfahrensschritte (a) und (b).

Wie zuvor geschildert, erfolgt in Verfahrensschritt (a) die Bereitstellung mindestens eines alkoholischen, insbesondere ethanolischen Pflanzenextraktes der vorgenannten Art.

Die Bereitstellung des oder der alkoholischen, insbesondere ethanolischen Pflanzenextrakte der vorgenannten Art, beispielsweise auf Basis von Thymian und/oder Primelwurzel, ist dem Fachmann als solches bekannt und erfolgt mit dem Fachmann an sich geläufigen Methoden, so dass es diesbezüglich keiner weitergehenden Ausführungen bedarf.

Erfindungsgemäß wird dabei in Verfahrensschritt (a) der mindestens eine alkoholische Pflanzenextrakt der vorgenannten Art mit einem Droge/Extrakt-Verhältnis im Bereich von 1 : 10 bis 10 : 1, insbesondere im Bereich von 5 : 1 bis 1 : 5, vorzugsweise im Bereich von 3 : 1 bis 1 : 4, bevorzugt im Bereich von 2 : 1 bis 1 : 3,5, ganz besonders bevorzugt im Bereich von 1 : 1 bis 1 : 3, bereitgestellt.

Das Droge/Extrakt-Verhältnis (DEV) gibt an, aus welcher Menge an eingesetzter Droge, z. B. Thymian, Primelwurzel etc., welche Menge Extrakt gewonnen wurde. Ein Droge/Extrakt-Verhältnis von z. B. 6 : 1 bedeutet, dass aus sechs Tonnen Droge eine Tonne Extrakt gewonnen wurde. Das Droge/Extrakt-Verhältnis gibt also an, wie viele Teile einer Arzneidroge für die Herstellung des Extraktäquivalents benötigt werden.

Die Verwendung von Extrakten mit streng definiertem Droge/Extrakt-Verhältnis (DEV) ist dahingehend relevant, dass die Qualität des Extraktes einen entscheidenden Einfluss auf die Gesamtqualität der pharmazeutischen Zubereitung hat. Ziel ist dabei, den Extrakt hinsichtlich der Wirkstoffkonzentration zu standardisieren, so dass auch die finale pharmazeutische Zubereitung qualitativ und quantitativ mit konstanten bzw. konstant guten Werten bezüglich der Wirk- und Inhaltsstoffe bereitgestellt werden kann.

Das erfindungsgemäß ausgewählte Droge/Extrakt-Verhältnis gewährleistet insbesondere eine ausreichende Wirkstoffkonzentration, um zu einem pharmazeutisch bzw. medizinisch wirksamen Endprodukt zu gelangen. Andererseits lässt sich jedoch ein Extrakt mit diesem Droge/Extrakt-Verhältnis ökonomisch und gut handhabbar herstellen. Dabei sind dem Fachmann die zur Herstellung erforderlichen Parameter, wie die Wahl des Auszugsmittels sowie der Extraktionsbedingungen, als solche bekannt. Bevorzugte Auszugsmittel sind dabei insbesondere Ethanol, aber auch andersartige Auszugsmittel, wie beispielsweise Mischungen von Ammoniaklösungen und glykolbasierten Lösungsmitteln, wie Glycerin. Sowohl Mischextrakte, zum Beispiel Mischextrakt von Thymian und Primelwurzel, als auch Einzelextrakte der vorgenannten Pflanzen bzw. Arzneidrogen werden mittels der Auswahl des Auszugsmittels sowie der Extraktionsbedingungen auf das zuvor angegebene Droge/Extrakt-Verhältnis eingestellt.

Was die stoffliche Zusammensetzung des in Verfahrensschritt (a) bereitgestellten alkoholischen Extrakts betrifft, so handelt es sich hierbei insbesondere um einen ethanolbasierten Pflanzenextrakt.

Insbesondere ist es erfindungsgemäß vorgesehen, dass in Verfahrensschritt (a) der mindestens eine alkoholische Pflanzenextrakt mit einem Gehalt an Alkohol, insbesondere Ethanol, von mehr als 10 Vol.-% und/oder mehr als 10 Gew.-%, insbesondere mehr als 20 Vol.-% und/oder mehr als 20 Gew.-%, besonders bevorzugt mehr als 30 Vol.-% und/oder mehr als 30 Gew.-%, bereitgestellt wird.

Dabei ist es erfindungsgemäß üblicherweise vorgesehen, dass in Verfahrensschritt (a) der mindestens eine alkoholische Pflanzenextrakt mit einem Gehalt an Alkohol, insbesondere Ethanol, im Bereich von 10 Vol.-% bis 99 Vol.-% und/oder im Bereich von 10 Gew.-% bis 99 Gew.-%, insbesondere im Bereich von 20 Vol.-% bis 95 Vol.% und/oder im Bereich von 20 Gew.-% bis 95 Gew.-%, besonders bevorzugt im Bereich von 30 Vol.-% bis 90 Vol.-% und/oder im Bereich von 30 Gew.-% bis 90 Gew.-%, bereitgestellt wird.

Der Alkoholgehalt in Extrakten von pflanzlichen Wirk- bzw. Inhaltsstoffen ist hinsichtlich der Effizienz der Extraktion von zentraler Bedeutung, da sich die pflanzlichen Wirk- bzw. Inhaltsstoffe, insbesondere apolare organische Verbindungen des pflanzlichen Sekundärmetabolismus, wie Carotinoide, oder phenolische Substanzen, wie Thymol, meist nur in organischen Lösungsmitteln, wie beispielsweise Ethanol oder Glycerin, jedoch nicht in wässrigen Lösungsmitteln effizient lösen. Sowohl Mischextrakte als auch Einzelextrakte, beispielsweise solche von Thymian und/oder Primelwurzeln, werden auf den definierten Alkoholgehalt eingestellt, um eine optimale Extraktion der Inhalts- bzw. Wirkstoffe sicherzustellen.

Bevorzugterweise erfolgt Verfahrensschritt (a) unter Bereitstellung einer Kombination von mindestens zwei alkoholischen Pflanzenextrakten, wobei bevorzugterweise jedoch beide Extrakte zunächst als Einzelextrakte bereitgestellt und erst nachfolgend gemischt bzw. kombiniert werden. Die Bereitstellung in Form von Einzelextrakten ist dahingehend vorteilhaft, dass sich durch die spezielle Wahl unterschiedlicher Auszugsmittel die Einzelextrakte jeweils mit höherer Effizienz herstellen lassen, als dies im Falle eines Mischextraktes der Fall ist.

Dies wird nachfolgend - rein beispielhaft - an einer Kombination von ThymianExtrakt einerseits und Primelwurzel-Extrakt andererseits erläutert: So kann beispielsweise die Herstellung von Thymianflüssigextrakt besonders effizient unter Verwendung von 10%iger Ammoniaklösung, 85%igem Glycerin, 90%igem Ethanol sowie Wasser in einem Verhältnis von 1 : 20 : 70 : 109 als Auszugsmittel erfolgen. Die Wirkstoffe anderer Pflanzen hingegen stellen aufgrund ihrer anderen chemischen Eigenschaften andere Anforderungen an das Extraktionsmittel. So lassen sich beispielsweise die Saponine der Primelwurzeln zur Herstellung eines Primelwurzelflüssigextraktes wesentlich effizienter z. B. unter ausschließlicher Verwendung von 70%igem Ethanol als Auszugsmittels extrahieren. Wie das Beispiel der Thymianextrakt/Primelwurzelextrakt-Kombination zeigt, wäre im Falle eines Mischextraktes eine optimale Extraktion kaum möglich, da dazu sehr unterschiedliche Auszugsmittel benötigt werden.

Ein weiterer Vorteil der Kombination von Einzelextrakten gegenüber Mischextrakten (d. h. Extrakten, bei denen Bestandteile von zwei oder mehr Arzneidrogen gemeinsam extrahiert werden) ist die Tatsache, dass die Wirkstoffkonzentration bei der Kombination von Einzelextrakten höher ist, da die Extraktion jeweils für den jeweiligen Wirkstoff bzw. die jeweilige Pflanze (Arzneidroge) optimiert durchgeführt werden kann.

In dem sich Verfahrensschritt (a) anschließenden Verfahrensschritt (b) des erfindungsgemäßen Verfahrens erfolgt - wie zuvor ausgeführt - eine Entfernung, vorzugsweise eine selektive Entfernung, des Alkohols, insbesondere des Ethanols, aus dem in Verfahrensschritt (a) bereitgestellten mindestens einen alkoholischen, insbesondere ethanolischen Pflanzenextrakt mittels Membranfiltration zum Erhalt einer im Wesentlichen alkoholfreien Zusammensetzung auf Basis mindestens eines Pflanzenextraktes mit sekretolytischer und/oder expektorationsfördernder Wirkung.

Was die eingesetzten Pflanzenextrakte als solche anbelangt, so werden in erfindungsgemäß bevorzugter Weise mindestens zwei verschiedene Pflanzenextrakte der vorgenannten Art in einer Zubereitung eingesetzt, wie beispielsweise Thymianund Primelwurzelextrakt. Denn verschiedene Wirkstoffe unterschiedlicher Pflanzenextrakte können sich in ihrem Effekt synergistisch ergänzen bzw. verstärken, wie es z. B. bei Thymian- und Primelwurzelextrakt der Fall ist: Thymol weist nämlich neben einem leicht expektorierenden Effekt auch hervorragende antimikrobielle bzw. antivirale sowie schmerzlindernde Eigenschaften auf, wohingegen Saponine stark schleimlösend wirken.

Was die erfindungsgemäß in Verfahrensschritt (b) durchgeführte Membranfiltration sowie die dazu verwendeten Membranfilter anbelangt, so ist diesbezüglich Folgendes auszuführen:
Der Oberbegriff der Membrantechnik bzw. Membranfiltration umfasst alle verfahrenstechnischen Maßnahmen zur Separation bzw. Trennung von Stoffgemischen unter Verwendung (semi)permeabler oder selektiv permeabler Membranen. In den Bereich der Membrantechnik bzw. Membranfiltration fallen unter anderem die Mikro-, Ultra- und Nanofiltration sowie die umgekehrte Osmose (Umkehrosmose bzw. Hyperfiltration) und die Dialyse. Neben Differenzen hinsichtlich der jeweils zugrundeliegenden physikalischen Prinzipien liegen die wesentlichen Unterscheidungsmerkmale der vorgenannten Verfahren in ihren Trenngrenzen.

Werden Partikel mit einer Größe im Bereich von 0,1 bis 10 µm abgetrennt, spricht man von einer so genannten Mikrofiltration. Liegt die Größe der abzutrennenden Partikel zwischen 0,01 und 0,1 µm, findet die so genannte Ultrafiltration Verwendung. Die so genannte Ultrafiltration wird im Prozessbereich überwiegend dynamisch durchgeführt und zur An- bzw. Abreicherung gelöster mittel- und hochmolekularer Stoffe, wie Enzyme, Hormone, Proteine, Antibiotika, Pyrogene, Viren oder Polysaccharide, verwendet. Die Ausschlussgrenze der so genannten Nanofiltration liegt im Bereich von 0,001 bis 0,01 µm. Die umgekehrte Osmose (Umkehrosmose bzw. Hyperfiltration) und die Dialyse ermöglichen mit Ausschlussgrenzen im Bereich von 0,0001 bis 0,001 µm die feinste Auftrennung. Nanofiltration, umgekehrte Osmose und Dialyse erlauben so auch die Abtrennung sehr kleiner Moleküle. Hinsichtlich der Antriebskraft ist die Dialyse von den übrigen vorgenannten Trennverfahren abzugrenzen, da sie überwiegend durch einen Konzentrationsgradienten angetrieben wird. Mikro-, Ultra- und Nanofiltration sowie die umgekehrte Osmose hingegen werden durch hydrostatische Druckdifferenzen zwischen den beiden durch die (semi)permeable bzw. selektiv permeable Membran getrennten Flüssigkeiten angetrieben. Je feiner die Ausschlussgrenze ist, desto höher ist der erforderliche Druck. Dies ist unter anderem darauf zurückzuführen, dass grobe Trennverfahren, wie Mikro- und Ultrafiltration, nach dem "Sieb"-Prinzip funktionieren, wohingegen die deutlich feinere Nanofiltration sowie die umgekehrte Osmose dem Lösungs-Diffusions-Modell folgen.

Für weitergehende Einzelheiten zu dem Begriff der Membranfiltration bzw. der Membranfiltertechnologie kann beispielsweise verwiesen werden auf Römpp Lexikon Chemie, Band 6, 10. Auflage, Georg Thieme Verlag, Stuttgart/New York, 1999, Seite 2584, Stichworte: *"Membranfilter", "Membranfiltration", "Ultrafiltration", "Mikrofiltration", "Dialyse"* und *"umgekehrte Osmose",* sowie die dort jeweils referierte Literatur, deren jeweiliger Inhalt hiermit durch Bezugnahme eingeschlossen ist.

Erfindungsgemäß wird in Verfahrensschritt (b) die Membranfiltration bevorzugt als Ultrafiltration, Nanofiltration, Umkehrosmose (Hyperfiltration) oder Dialyse, vorzugsweise als Umkehrosmose (Hyperfiltration) oder Dialyse, bevorzugt als Umkehrosmose (Hyperfiltration), durchgeführt.

Die Dialyse ist - wie bereits zuvor erwähnt - ein konzentrationsgetriebener Membranprozess, mit dessen Hilfe sehr kleine Teilchen, wie beispielsweise Ionen oder kleine Moleküle (z. B. Ethanol), aus Lösungen entfernt werden können. Dabei erfolgt die Abtrennung mittels Diffusion der Teilchen aus der Dispersion in ein laufend erneuertes reines Lösungsmittel, wobei es sich bei dem Lösungsmittel im Allgemeinen um Wasser handelt. Zur Dialyse werden (semi)permeable bzw. selektiv permeable Membranen eingesetzt, welche keine durchgehenden Poren besitzen, da der Transport bei diesem Membranprozess ebenfalls dem Lösungs-Diffusions-Modell folgt. Bei der Dialyse entspricht die Diffusionsrichtung der natürlichen Osmose.

Die Umkehrosmose ist ein physikalisches Verfahren zur Aufkonzentrierung von in Flüssigkeiten gelösten Stoffen, bei dem mit hydrostatischem Druck der natürliche Osmose-Prozess umgekehrt wird. Das Medium, in dem die Konzentration eines bestimmten Stoffes verringert werden soll, ist durch eine halbdurchlässige (semipermeable) bzw. selektiv permeable Membran von dem Medium getrennt, in dem die Konzentration erhöht werden soll. Dieses wird einem Druck ausgesetzt, der höher sein muss als der Druck, der durch das osmotische Verlangen zum Konzentrationsausgleich entsteht. Dadurch können die Moleküle des Lösungsmittels gegen ihre "natürliche" osmotische Ausbreitungsrichtung in den Bereich wandern, in dem die gelösten Stoffe bereits geringer konzentriert sind. Diese Druckdifferenz zwischen beiden Flüssigkeiten kann bis zu 200 bar betragen. Im Gegensatz zu herkömmlichen Membranfiltern verfügen Osmosemembranen nicht über durchgehende Poren, da der Transport nicht wie bei der Mikro- und Ultrafiltration über einen druckbetriebenen "Siebeffekt" erfolgt, sondern vielmehr dem Lösungs-Diffusions-Modell folgt.

Im Rahmen der vorliegenden Erfindung wird die Membranfiltration, vorzugsweise Umkehrosmose (Hyperfiltration), mit einer Druckdifferenz und/oder einem Druckgradienten und/oder einer hydrostatischen Druckdifferenz im Bereich von 10 bis 100 bar, insbesondere 20 bis 90 bar, vorzugsweise jedoch 30 bis 80 bar durchgeführt.

Was den Verfahrensschritt (b) weiterhin betrifft, so wird die Membranfiltration mittels mindestens eines Membranfilters durchführt. Der Membranfilter dient einerseits der vorzugsweise selektiven Zurückhaltung der erwünschen Substanzen, andererseits ist er vorzugsweise selektiv permeabel für die abzutrennenden Substanzen (d. h. insbesondere Ethanol).

Um eine Selektivität des Membranfilters zu erreichen, wie es erfindungsgemäß erfolgt, so spielen die Eigenschaften des Filters eine tragende Rolle.

Was das Material des erfindungsgemäß in Verfahrensschritt (b) eingesetzten Membranfilters betrifft, so kann dieses aus anorganischen oder organischen Materialien, insbesondere jedoch aus organischen Materialien, bevorzugt polymeren organischen, Materialien ausgebildet sein. Insbesondere kann das Membranmaterial aus der Gruppe von (i) fluorierten und perfluorierten organischen Polymeren, insbesondere fluorierten und perfluorierten Olefinen, vorzugsweise Polytetrafluorethylenen; (ii) Materialien auf Basis von Cellulose oder Cellulosederivaten, insbesondere Celluloseacetaten und regenierter Cellulose; (iii) sulfonierten organischen Polymeren, insbesondere Polysulfonen und Polyethersulfonen; (iv) Polyolefinen, insbesondere Polyethylenen und Polypropylenen; (v) Polyamiden; (vi) Polyestern sowie deren Kombinationen, ausgewählt werden. Polymerbasierte Membranfilter sind besonders leicht in der Handhabung und zudem äußerst kostengünstig. Weiterhin erlauben sie die effiziente und vorzugsweise selektive Abtrennung des Alkohols, insbesondere des Ethanols.

Weiterhin kann das erfindungsgemäß in Verfahrensschritt (b) eingesetzte Membranfilter einschichtig oder mehrschichtig, insbesondere als Kompositmaterial, ausgebildet sein. Kompositmaterialien erhöhen einerseits die Selektivität, andererseits wird auch die Stabilität der Membran verbessert, was insbesondere im Hinblick auf den erfindungsgemäß eingesetzten hohen Druck relevant ist.

Zudem kann das Membranfilter faserverstärkt ausgebildet sein. Durch eine Faserverstärkung kann die Stabilität noch weiter verbessert werden.

Was die Ausgestaltung der Poren des erfindungsgemäß in Verfahrensschritt (b) eingesetzten Membranfilters anbelangt, so weist dieses vorteilhafterweise eine Porengröße von höchstens 0,001 µm, insbesondere im Bereich von 0,0001 bis 0,001 µm, auf.

Auch weist das erfindungsgemäß in Verfahrensschritt (b) eingesetzte Membranfilter eine selektive Permeabilität und/oder Durchlässigkeit auf. Diesbezüglich ist das Membranfilter insbesondere selektiv durchlässig in Bezug auf Alkohol, insbesondere Ethanol, ausgebildet. Mit anderen Worten ist das Membranfilter durchlässig, vorzugsweise selektiv durchlässig, in Bezug auf Alkohol, insbesondere Ethanol, ausgebildet. Für die Inhalts- und Wirkstoffe der eingesetzten Pflanzenextrakte hingegen ist das eingesetzte Membranfilter vorzugsweise impermeabel bzw. zumindest im Wesentlichen impermeabel.

Zudem ist das Membranfilter vorteilhafterweise undurchlässig in Bezug auf Wasser ausgebildet ist.

Durch die vorgenannte Ausgestaltung des Membranfilters kann insbesondere erreicht werden, dass die Entfernung des Alkohols, insbesondere des Ethanols, in einer Weise erfolgt, dass die medizinisch wirksamen Inhalts- bzw. Wirkstoffe des mindestens einen Pflanzenextraktes mit sekretolytischer und/oder expektorationsfördernder Wirkung durch die Membranfiltration zumindest weitestgehend nicht entfernt werden und der Alkohol, insbesondere der Ethanol, selektiv entfernt wird.

Was den erfindungsgemäßen Verfahrensschritt (b) weiterhin anbelangt, so wird die Membranfiltration insbesondere bei Temperaturen im Bereich von 5 bis 80 °C, insbesondere 10 bis 70 °C, vorzugsweise 15 bis 60 °C, besonders bevorzugt 20 bis 50 °C, durchgeführt. Dies ist insbesondere dahingehend relevant, dass durch die gezielte Auswahl des Temperaturbereichs ein Substanzverlust der Inhalts- und Wirkstoffe der Pflanzenextrakte vermieden kann, da diese so weder in ihrer chemischen Stabilität beeinträchtigt werden noch aufgrund zu hoher Temperaturen evaporieren. Der maximale Substanzverlust bei der Membranfiltration beträgt insbesondere höchstens 10 Gew.-%, bevorzugterweise jedoch nicht mehr als 5 Gew.-%, besonders bevorzugt nicht mehr als 1 Gew.-%, ganz besonders bevorzugt nicht mehr als 0,5 Gew.-%, noch mehr bevorzugt nicht mehr als 0,1 Gew.-%, bezogen auf die Wirk- bzw. Inhaltsstoffe des mindestens einen Pflanzenextrakts.

Erfindungsgemäß kann die Membranfiltration gemäß Verfahrensschritt (b) kontinuierlich oder diskontinuierlich, vorzugsweise kontinuierlich, durchgeführt werden. Mittels einer kontinuierlichen Membranfiltration läuft das Verfahren noch deutlich zeitund kostenreduzierter ab, da zusätzliche Verfahrensschritte eingespart werden können. Auch wird durch eine kontinuierliche Verfahrensführung das Risiko von Kontaminationen oder Verunreinigungen reduziert.

Auch kann der Verfahrensschritt (b) durch die Auswahl bestimmter Prozessparameter bzw. Prozesssysteme hinsichtlich der Verfahrensökonomie vorteilhaft ausgestaltet werden:
So kann die Membranfiltration erfindungsgemäß in einem offenen oder geschlossenen System, vorzugsweise jedoch in einem geschlossenen System, durchgeführt werden. Die Durchführung im geschlossenen System ist insbesondere im Hinblick auf die Vermeidung von Substanzverlusten der Wirk- und Inhaltsstoffe einerseits, aber auch auf die Vermeidung von Kontaminationen andererseits von besonderer Bedeutung. Insbesondere Pflanzenextrakte weisen häufig volatile Wirk- und Inhaltsstoffe, wie beispielsweise ätherische Öle etc., auf, welche über offene Systeme leicht aus dem Extrakt diffundieren und somit zu Substanzverlusten führen können.

Was das erfindungsgemäße Verfahren weiterhin anbelangt, so wird dem in Verfahrensschritt (a) bereitgestellten alkoholischen, insbesondere ethanolischen Pflanzenextrakt vor der Durchführung der Membranfiltration gemäß Verfahrensschritt (b) üblicherweise Wasser zugesetzt. Dabei kann der Alkoholanteil des Pflanzenextraktes insbesondere auf einen Wert von höchstens 40 Vol.-% und/oder 40 Gew.-%, insbesondere höchstens 35 Vol.-% und/oder 35 Gew.-%, vorzugsweise höchstens 30 Vol.-% und/oder 30 Gew.-%, eingestellt werden. Durch die Herabsetzung des relativen Alkoholanteils in den Pflanzenextrakten bereits vor Durchführung der Membranfiltration verläuft die Entfernung des Alkohols, insbesondere des Ethanols, mittels anschließender Membranfiltration wesentlich effizienter. Weiterhin wird durch den herabgesetzten Alkoholanteil das Membranfilter geschont, da das Material durch hohe Alkohol-Konzentrationen, insbesondere von Ethanol, beeinträchtigt bzw. geschädigt werden kann bzw. schneller Verschleißerscheinungen aufweisen kann.

Erfindungsgemäß wird dem in Verfahrensschritt (a) bereitgestellten alkoholischen, insbesondere ethanolischen Pflanzenextrakt vor der Durchführung der Membranfiltration mindestens ein Dispergier- oder Netzmittel, vorzugsweise ein Lösungsvermittler, zugesetzt. Mit anderen Worten wird die Membranfiltration in Gegenwart mindestens eines Dispergiermittels bzw. Netzmittels, vorzugsweise Lösungsvermittlers, durchgeführt. Dabei ist das Dispergier- oder Netzmittel ausgewählt aus der Gruppe von organischen Ölen, Fetten und Fettsäuren, Lecithinen, Glyceriden sowie deren Kombinationen. Dabei wird das mindestens eine Dispergier- oder Netzmittel in Mengen von 0,1 bis 30 Gew.-%, insbesondere 0,5 bis 20 Gew.-%, vorzugsweise 1 bis 15 Gew.-%, bezogen auf die der Membranfiltration zu unterziehende Gesamtmischung aus alkoholischem Pflanzenextrakt, Dispergier- oder Netzmittel, Wasser und gegebenenfalls weiteren Inhaltsstoffen, eingesetzt. Die Verwendung eines Dispergier- bzw. Netzmittels, vorzugsweise eines Lösungsvermittlers, ist insbesondere im Hinblick auf die Vermeidung von Substanzverlusten während der Membranfiltration von zentraler Bedeutung, da die Wirk- und Inhaltsstoffe ansonsten gegebenenfalls zu Präzipitationen neigen können oder über die Bildung eines Präzipitats, insbesondere Filterkuchens, verlorengehen können.

Was das erfindungsgemäße Verfahren weiterhin betrifft, so kann es vorteilhafterweise vorgesehen sein, dass in Verfahrensschritt (b) die Membranfiltration einstufig oder mehrstufig, vorzugsweise mehrstufig, durchgeführt wird. Durch ein mehrstufiges Verfahren kann eine deutlich effizientere Entfernung des Alkohols, insbesondere des Ethanols, aus den Pflanzenextrakten erfolgen.

Im Hinblick auf eine mehrstufige Durchführung der Membranfiltration in Verfahrensschritt (b), so erfolgt diese vorzugsweise in mindestens zwei, insbesondere mindestens drei aufeinanderfolgenden Stufen (Teilschritten), besonders bevorzugt in drei bis zehn aufeinanderfolgenden Stufen (Teilschritten). Dabei wird die Membranfiltration in jeder Stufe derart durchgeführt, dass der Alkoholgehalt, insbesondere Ethanolgehalt, bei jeder Stufe um mindestens 20 %, insbesondere um mindestens 30 %, vorzugsweise um mindestens 40 %, bezogen auf den Ausgangsalkoholgehalt zu Beginn der jeweiligen Stufe, reduziert wird. (Zur Erläuterung: Eine Reduktion um mindestens 20 % z. B. bei einem Ethanol-Ausgangsgehalt von 70 Vol.% bedeutet also eine Reduktion um mindestens 14 Vol.-%, d. h. auf mindestens 56 Vol.-%, in dieser Stufe). Diesbezüglich kann zu Beginn jeder Stufe der betreffenden Zusammensetzung vor der Durchführung der Membranfiltration Wasser zugesetzt werden.

Üblicherweise wird die in Verfahrensschritt (b) resultierende, im Wesentlichen alkoholfreie Zusammensetzung auf Basis mindestens eines Pflanzenextraktes mit sekretolytischer und/oder expektorationsfördernder Wirkung als Retentat der Membranfiltration erhalten. Der zumindest im Wesentlichen vollständig entfernte Alkohol, insbesondere Ethanol, hingegen liegt im Permeat der Membranfiltration vor.

Im Allgemeinen entspricht der nach Verfahrensschritt (b) resultierende Gehalt an Inhalts- bzw. Wirkstoffen im Pflanzenextrakt gemäß allen erfindungsgemäßen Ausführungsformen zumindest im Wesentlichen dem Gehalt an Inhalts- bzw. Wirkstoffen, wie er in der alkoholischen Ausgangszusammensetzung enthalten ist. Dies konnte von der Anmelderin experimentell nachgewiesen werden, insbesondere durch chromatographische Nachweise.

Was das erfindungsgemäße Verfahren weiterhin betrifft, so kann sich Verfahrensschritt (b) ein Verfahrensschritt (c) anschließen, bei dem die in Verfahrensschritt (b) resultierende, zumindest im Wesentlichen alkoholfreie Zusammensetzung auf Basis mindestens eines Pflanzenextraktes mit sekretolytischer und/oder expektorationsfördernder Wirkung auf eine gewünschte finale Zusammensetzung, insbesondere in einer für die perorale Applikation hergerichteten Form, eingestellt wird.

Insbesondere kann es erfindungsgemäß vorgesehen sein, dass in diesem Verfahrensschritt (c) der in Verfahrensschritt (b) resultierenden, im Wesentlichen alkoholfreien Zusammensetzung auf Basis mindestens eines Pflanzenextraktes mit sekretolytischer und/oder expektorationsfördernder Wirkung Wasser und/oder ein wässriger Exzipient und/oder gegebenenfalls mindestens ein weiterer Inhaltsstoff zugesetzt wird bzw. werden.

Um zu gewährleisten, dass die aus Verfahrensschritt (b) resultierende, im Wesentlichen alkoholfreie Zusammensetzung auch nach der Zugabe eines wässrigen Exzipienten im Wesentlichen alkoholfrei bleibt und insbesondere auf einen wie zuvor spezifizierten Alkoholgehalt eingestellt werden kann, so ist auch der wässrige Exzipient zumindest im Wesentlichen alkoholfrei ausgebildet. Wie im Folgenden noch weiter ausgeführt wird, handelt es sich bei dem wässrigen Exzipienten bevorzugterweise um eine Mischung aus Wasser sowie mindestens einem weiteren Inhaltsstoff, wie z. B. gegebenenfalls Saccharose und gegebenenfalls einem Sirup, welcher vorzugsweise Glucose und Fructose, bevorzugt in etwa gleichem Stoffmengenanteil (Invert-Sirup), enthält.

Wie bereits erwähnt, enthält der erfindungsgemäß in Verfahrenschritt (c) eingesetzte wässrige Exzipient, neben Wasser mindestens ein(en) vorzugsweise flüssigen Zucker, Zuckeraustauschstoff und/oder Kohlenhydrat, bevorzugt auf Basis einer Mischung von Saccharose und einem Sirup, insbesondere einem Sirup mit Glucose und Fructose, vorzugsweise in etwa gleichem Stoffmengenanteil (so genannter Invert-Sirup). Neben einer effizienten Aufnahme der Wirk- und Inhaltsstoffe der im Wesentlichen alkoholfreien Pflanzenextrakte verleiht insbesondere ein wässriges Zucker- bzw. Sirupgemisch der Zusammensetzung auch angenehme organoleptische Eigenschaften, insbesondere einen angenehmen süßen Geschmack, der die starken Aromen der Inhalts- bzw. Wirkstoffe kompensiert bzw. hierzu kompatibel ist.

Der Begriff "Zucker" wird im Rahmen der vorliegenden Erfindung insbesondere für süßschmeckende Saccharide (z. B. Einfach- und Doppelzucker), insbesondere jedoch für den Doppelzucker Saccharose, welcher dem gewöhnlichen Haushaltszucker entspricht, verwendet. Der vorzugsweise flüssige Zucker verleiht der finalen pharmazeutischen Zusammensetzung bereits nach Aufnahme der im Wesentlichen alkoholfreien Pflanzenextrakte eine angenehme Süße, was das Erfordernis von weiteren Zusatzstoffen zur Geschmacksmodifizierung vermindert bzw. entbehrlich macht.

Unter dem Begriff "Zuckeraustauschstoffe", wie er erfindungsgemäß verwendet wird, versteht man eine Sammelbezeichnung für Stoffe, welche anstelle von Saccharose zur Süßung der pharmazeutischen Zusammensetzung dienen können. Der physiologische Vorteil der Zuckeraustauschstoffe im Vergleich zur Saccharose liegt in der insulinunabhängigen Metabolisierung (Diabetiker) und in der zum Teil verminderten kariogenen Wirkung. Für weitergehende Einzelheiten zu dem Begriff der Zuckeraustauschstoffe kann beispielsweise verwiesen werden auf Römpp Lexikon Chemie, Band 6, 10. Auflage, Georg Thieme Verlag, Stuttgart/New York, 1999, Seite 5098 bis 5100, Stichwort: "Zuckeraustauschstoffe", und auf Römpp Lexikon Lebensmittelchemie, 9. Auflage, Georg Thieme Verlag, Stuttgart/New York, Seite 955, Stichwort: "Zuckeraustauschstoffe", sowie die dort jeweils referierte Literatur, deren jeweiliger Inhalt hiermit durch Bezugnahme eingeschlossen ist.

Auch ist es möglich, dass der wässrige Exzipient neben Wasser mindestens ein weiteres Additiv, insbesondere aus der Gruppe von Dispergier- oder Netzmitteln und/oder oberflächenaktiven Substanzen, Konservierungsmitteln, Stabilisatoren, geschmacks- und/oder geruchsmodifizierenden Substanzen, Rheologiemodifikatoren, Verdickungsmitteln, Farbstoffen, Puffern, pH-Stellmitteln, Emulgatoren, Suspensionsmitteln oder deren Mischungen, enthält, so dass die Zusammensetzung in ihren Eigenschaften nahezu beliebig modifizierbar ist.

Zudem enthält der in Verfahrenschritt (c) eingesetzte wässrige Exzipient Wasser in Mengen von 5 bis 95 Gew.-%, insbesondere 10 bis 90 Gew.-%, vorzugsweise 20 bis 80 Gew.-%, bezogen auf den Exzipienten.

Was den in Verfahrenschritt (c) einsetzbaren weiteren Inhaltsstoff betrifft, so kann dieser insbesondere aus der Gruppe von vorzugsweise flüssigen Zuckern, Zuckeraustauschstoffen und/oder Kohlenhydraten, bevorzugt auf Basis einer Mischung von Saccharose und einem Sirup, insbesondere einem Sirup mit Glucose und Fructose, vorzugsweise in etwa gleichem Stoffmengenanteil (Invert-Sirup), und/oder Additiven, insbesondere Dispergier- oder Netzmitteln und/oder oberflächenaktiven Substanzen, Konservierungsmitteln, Stabilisatoren, geschmacksund/oder geruchsmodifizierenden Substanzen, Rheologiemodifikatoren, Verdickungsmitteln, Farbstoffen, Puffern, pH-Stellmitteln, Emulgatoren, Suspensionsmitteln oder deren Mischungen ausgewählt werden. Durch den weiteren Inhaltsstoff kann die Zusammensetzung in ihren Eigenschaften noch weiter modifiziert bzw. angepasst werden.

Insbesondere ist es erfindungsgemäß vorgesehen, dass die Zusammensetzung nach der vorliegenden Erfindung zumindest im Wesentlichen alkoholfrei ausgebildet ist. Zu diesem Zweck ist es erfindungsgemäß vorgesehen, dass die Zusammensetzung in Verfahrensschritt (b) und/oder in Verfahrensschritt (c) auf einen Alkoholgehalt, insbesondere einen Ethanolgehalt, von weniger als 2 Vol.-%, insbesondere weniger als 1,0 Vol-%, vorzugsweise weniger als 0,85 Vol.-%, besonders bevorzugt weniger als 0,5 Vol.-%, ganz besonders bevorzugt weniger als 0,25 Vol.%, noch mehr bevorzugt weniger als 0,1 Vol.-%, am meisten bevorzugt auf etwa 0 Vol.-%, eingestellt ist. Die Einstellung des Alkoholgehalts erfolgt zum einen dadurch, dass in Verfahrensschritt (b) der Alkohol zumindest im Wesentlichen entfernt wird, und zum anderen dadurch, dass in dem optionalen Verfahrensschritt (c) Wasser und/oder ein zumindest im Wesentlichen alkoholfreier Exzipient zum Einsatz kommen kann.

Zudem wird die pharmazeutische Zusammensetzung erfindungsgemäß in Verfahrensschritt (b) und/oder Verfahrensschritt (c) auf eine unter Normalbedingungen, insbesondere bei einer Temperatur von 25 °C und unter einem Druck von 1,01325 bar, flüssige und/oder fließfähige Viskosität oder Konsistenz eingestellt, um eine flüssige Dosierungsform für die orale Applikation zu gewährleisten.

Weiterer erfindungsgemäßer Gegenstand - gemäß einem **zweiten** Aspekt der vorliegenden Erfindung - ist eine Zusammensetzung auf Basis mindestens eines Pflanzenextraktes mit sekretolytischer und/oder expektorationsfördernder Wirkung, insbesondere eine pharmazeutische Zubereitung, welche sich insbesondere zur prophylaktischen und/oder therapeutischen Behandlung von Atemwegserkrankungen, Erkältungskrankheiten sowie grippalen Infekten und damit verbundenen Symptomen eignet und durch ein Verfahren erhältlich ist, wie es zuvor beschrieben wurde.

Insbesondere ist Gegenstand der vorliegenden Erfindung gemäß diesem Erfindungsaspekt eine nach dem zuvor beschriebenen Verfahren erhältliche Zusammensetzung, insbesondere eine pharmazeutische Zubereitung, welche sich vorzugsweise zur prophylaktischen und/oder therapeutischen Behandlung von Atemwegserkrankungen, Erkältungskrankheiten sowie grippalen Infekten und damit verbundenen Symptomen eignet, wobei die Zusammensetzung mindestens einen Pflanzenextrakt mit sekretolytischer und/oder expektorationsfördernder Wirkung enthält und in Form einer zumindest im Wesentlichen alkoholfreien Zubereitung vorliegt, wobei die Zusammensetzung einen Alkoholgehalt, insbesondere einen Ethanolgehalt, von weniger als 2 Vol.-%, aufweist, wobei die Zusammensetzung mindestens ein Dispergier- oder Netzmittel, vorzugsweise Lösungsvermittler, enthält, wobei das Dispergier- oder Netzmittel ausgewählt ist aus der Gruppe von organischen Ölen, Fetten und Fettsäuren, Lecithinen, Glyceriden sowie deren Kombinationen und wobei der mindestens eine Pflanzenextrakt mit sekretolytischer und/oder expektorationsfördernder Wirkung ausgewählt ist aus der Gruppe von Thymian-Extrakt *(Thymus vulgaris),* Primelwurzel-Extrakt *(Primula),* Efeu-Extrakt (*Hedera helix*)*,* Holunder-Extrakt (*Sambucus nigra*)*,* Fenchel-Extrakt (*Foeniculum vulgare*), Huflattich-Extrakt (*Tussilago farfara*)*,* Anis-Extrakt (*Pimpinella anisum*) und Tüpfelfarn-Extrakt (*Polypodium vulgare*) sowie Mischungen und Kombinationen von mindestens zwei der vorgenannten Extrakte, wobei der Pflanzenextrakt ein Droge/Extrakt-Verhältnis im Bereich von 1 : 10 bis 10 : 1 aufweist.

Hinsichtlich des Begriffs "pharmazeutische Zusammensetzung" - synonym bisweilen auch als "pharmazeutische Zubereitung" bezeichnet - ist zu erwähnen, dass dieser im Rahmen der vorliegenden Erfindung sehr weit zu verstehen ist und nicht nur Arzneimittel, Medikamente, Pharmaka oder sonstige Medizinprodukte, welche laut Arzneimittelgesetz zur Diagnostik bzw. als Therapeutika bestimmt sind, bezeichnet, sondern darüber hinaus auch Nahrungsergänzungsmittel oder nicht unter das Arzneimittelgesetz fallende naturheilkundliche bzw. phytopharmazeutische Produkte, Zubereitungen oder Zusammensetzungen. Diesbezüglich kann auf obige Ausführungen verwiesen werden.

Im Rahmen der vorliegenden Erfindung wird, wie zuvor geschildert, eine zumindest im Wesentlichen alkoholfreie Zusammensetzung der vorgenannten Art bereitgestellt.

In diesem Zusammenhang kann der gegebenenfalls vorhandene Restalkoholgehalt in der erfindungsgemäßen Zusammensetzung in gewissen Grenzen variieren.

Erfindungsgemäß ist es vorgesehen, dass die Zusammensetzung einen Alkoholgehalt, insbesondere einen Ethanolgehalt, von weniger als 2 Vol.-%, insbesondere weniger als 1,0 Vol.-%, vorzugsweise weniger als 0,85 Vol.-%, besonders bevorzugt weniger als 0,5 Vol.-%, ganz besonders bevorzugt weniger als 0,25 Vol.-%, noch mehr bevorzugt weniger als 0,1 Vol.-%, am meisten bevorzugt von etwa 0 Vol.-%, aufweist.

Insbesondere Zusammensetzungen mit einem Alkoholgehalt, welcher geringer als 0,05 g Ethanol pro Einzeldosis bzw. geringer als 5.000 ppm oder 5 g/kg als Konzentration im finalen Produkt ist, können auch zur Anwendung bei Säuglingen, Kleinkindern und anderen Personengruppen mit Kontraindikationen bedenkenlos eingesetzt werden.

Weiterhin ist die erfindungsgemäße Zusammensetzung bevorzugt dergestalt, dass sie unter Normalbedingungen, insbesondere bei einer Temperatur von 25 °C und unter einem Druck von 1,01325 bar, flüssig und/oder fließfähig ist.

Was den mindestens einen Pflanzenextrakt mit sekretolytischer und/oder expektorationsfördernder Wirkung in der Zusammensetzung angeht, so ist dieser aus der Gruppe von Thymian-Extrakt, Primelwurzel-Extrakt, Efeu-Extrakt, Holunder-Extrakt, Fenchel-Extrakt, Huflattich-Extrakt, Anis-Extrakt und Tüpfelfarn-Extrakt sowie Mischungen und Kombinationen von mindestens zwei der vorgenannten Extrakte ausgewählt.

Erfindungsgemäß bevorzugt ist eine erfindungsgemäße Zusammensetzung, welche mindestens zwei der vorgenannten Pflanzenextrakte enthält. Auf diese Weise lässt sich eine Wirkungssteigerung durch Kombinationen unterschiedlicher Wirk- bzw. Inhaltsstoffe erreichen.

Eine erfindungsgemäß besonders bevorzugte Kombination von Pflanzenextraken in der erfindungsgemäßen Zusammensetzung kann zum Beispiel mindestens einen Extrakt von Thymian und mindestens einen Extrakt von Primelwurzel in Kombination oder aber einen Thymian/Primelwurzel-Mischextrakt umfassen; dies ist mit den vorgenannten Vorteilen verbunden, insbesondere aufgrund der vorteilhaften Kombination der unterschiedlichen Inhaltsstoffe dieser beiden Drogen.

Das Droge/Extrakt-Verhältnis des mindestens einen eingesetzten Pflanzenextraktes kann grundsätzlich in weiten Bereichen variieren. Erfindungsgemäß ist es vorgesehen, dass der mindestens eine Pflanzenextrakt ein Droge/Extrakt-Verhältnis im Bereich von 1 : 10 bis 10 : 1, insbesondere im Bereich von 5 : 1 bis 1 : 5, vorzugsweise im Bereich von 3 : 1 bis 1 : 4, bevorzugt im Bereich von 2 : 1 bis 1 : 3,5, ganz besonders bevorzugt im Bereich von 1 : 1 bis 1 : 3, aufweist. Wie zuvor geschildert, gewährleisten die vorgenannten Bereiche für das Droge/Extrakt-Verhältnis eine ausreichende Wirkeffizienz einerseits und eine ökonomische Verfügbarkeit andererseits.

Die Menge an Pflanzenextrakt in der erfindungsgemäßen Zusammensetzung kann in weiten Bereichen variieren. So enthält die Zusammensetzung den mindestens einen Pflanzenextrakt, berechnet als Trockenextrakt und bezogen auf die Zusammensetzung, in Mengen von 0,001 bis 50 Gew.-%, insbesondere 0,01 bis 30 Gew.-%, vorzugsweise 0,05 bis 20 Gew.-%, besonders bevorzugt 0,1 bis 10 Gew.-%. Die vorgenannten Mengen gewährleisten einerseits eine ausreichende Wirksamkeit und andererseits eine gute ökonomische Verfügbarkeit.

Wie zuvor geschildert, ist die erfindungsgemäße Zusammensetzung zumindest im Wesentlichen alkoholfrei ausgebildet.

Gemäß einer bevorzugten Ausführungsform ist die erfindungsgemäße Zusammensetzung wässrig basiert bzw. als wässrige Zubereitung formuliert bzw. umfasst einen wässrigen Exzipienten.

Die Menge an Wasser in der erfindungsgemäßen Zusammensetzung kann in weiten Bereichen variieren. Diesbezüglich kann es erfindungsgemäß vorgesehen sein, dass die Zusammensetzung Wasser, insbesondere in Mengen von 5 bis 99 Gew.-%, insbesondere 7,5 bis 70 Gew.-%, vorzugsweise 10 bis 50 Gew.-%, besonders bevorzugt 15 bis 40 Gew.-%, enthält, bezogen auf die Zusammensetzung. Die vorgenannten Wassermengen gewährleisten einerseits eine gute Formulierbarkeit der erfindungsgemäßen Zusammensetzung als flüssige bzw. fließfähige Zubereitung und andererseits eine gute orale Applizierbarkeit sowie darüber hinaus eine zumindest im Wesentlichen vollständige Solubilisierung aller Wirk- und Inhaltsstoffe.

Abgesehen von Wasser selbst kann der wässrige Exzipient aber auch noch andere Inhaltsstoffe, wie z. B. Zucker und/oder Zuckeraustauschstoffe und/oder Additive bzw. Hilfsstoffe etc., enthalten, wie nachfolgend noch im Detail geschildert.

Gemäß einer besonderen Ausführungsform kann es beispielsweise erfindungsgemäß vorgesehen sein, dass die Zusammensetzung - neben Wasser und dem mindestens einen Pflanzenextrakt - bzw. der wässrige Exzipient mindestens einen vorzugsweise flüssigen Zucker und/oder Zuckeraustauschstoff, bevorzugt auf Basis einer Mischung von Saccharose und einem Sirup, insbesondere einem Sirup mit Glucose und Fructose, vorzugsweise in etwa gleichem Stoffmengenanteil (Invert-Sirup), aufweist. Dabei kann die erfindungsgemäße Zusammensetzung den mindestens einen Zucker und/oder Zuckeraustauschstoff insbesondere in Mengen von 5 bis 95 Gew.-%, insbesondere 10 bis 90 Gew.-%, vorzugsweise 20 bis 90 %, besonders bevorzugt 40 bis 85 Gew.-%, ganz besonders bevorzugt 60 bis 85 Gew.-%, enthalten, bezogen auf die Zusammensetzung. Hierbei sollten die Mengenverhältnisse insbesondere derart eingestellt sein, dass die erfindungsgemäße Zusammensetzung trotz des Vorhandenseins von Zucker(n) und/oder Zuckeraustauschstoff(en) oder dergleichen unter Normalbedingungen, d. h. bei einer Temperatur von 25 °C und einem Druck von 1,01325 bar, flüssig bzw. fließfähig ist, so dass eine flüssige Dosierungsform zur oralen Applikation realisiert wird.

Erfindungsgemäß enthält die Zusammensetzung mindestens ein Additiv, welches ausgewählt ist aus der Gruppe von Dispergier- oder Netzmitteln, vorzugsweise Lösevermittlern. Darüber hinaus kann die Zusammensetzung auch Additive aus der Gruppe von Konservierungsmitteln, Stabilisatoren, geschmacks- und/oder geruchsmodifizierenden Substanzen, Rheologiemodifikatoren, Verdickungsmitteln, Farbstoffen, Puffern, pH-Stellmitteln, Emulgatoren, Suspensionsmitteln oder deren Mischungen enthalten. Das Additiv kann dabei insbesondere in Mengen von 0,001 bis 30 Gew.-%, insbesondere 0,01 bis 20 Gew.-%, vorzugsweise 0,05 bis 10 %, besonders bevorzugt 0,1 bis 5 Gew.-%, bezogen auf die Zusammensetzung, enthalten sein.

Eine erfindungsgemäß bevorzugte Zusammensetzung, insbesondere pharmazeutische Zubereitung, vorzugsweise zur prophylaktischen und/oder therapeutischen Behandlung von Atemwegserkrankungen, Erkältungskrankheiten sowie grippalen Infekten und damit verbundenen Symptomen ist insbesondere wie zuvor definiert ausgebildet, wobei die Zusammensetzung in Form einer zumindest im Wesentlichen alkoholfreien Zubereitung vorliegt und mindestens die folgenden Inhaltsstoffe enthält:
- mindestens einen Pflanzenextrakt mit sekretolytischer und/oder expektorationsfördernder Wirkung, jeweils mit einem Droge/Extrakt-Verhältnis im Bereich von 1 : 10 bis 10 : 1, insbesondere im Bereich von 5 : 1 bis 1 : 5, vorzugsweise im Bereich von 3 : 1 bis 1 : 4, bevorzugt im Bereich von 2 : 1 bis 1 : 3,5, ganz besonders bevorzugt im Bereich von 1 : 1 bis 1 : 3, insbesondere wobei die Zusammensetzung den mindestens einen Pflanzenextrakt, berechnet als Trockenextrakt, in Mengen von 0,001 bis 50 Gew.-%, insbesondere 0,01 bis 30 Gew.-%, vorzugsweise 0,05 bis 20 Gew.-%, besonders bevorzugt 0,1 bis 10 Gew.-%, enthält, und wobei der mindestens eine Pflanzenextrakt mit sekretolytischer und/oder expektorationsfördernder Wirkung ausgewählt ist aus der Gruppe von Thymian-Extrakt *(Thymus vulgaris),* Primelwurzel-Extrakt *(Primula),* Efeu-Extrakt (*Hedera helix*), Holunder-Extrakt (*Sambuca nigra*)*,* Fenchel-Extrakt (*Foeniculum vulgare*), Huflattich-Extrakt (*Tussilago farfara*), Anis-Extrakt (*Pimpinella anisum*) und Tüpfelfarn-Extrakt (*Polypodium vulgare*) sowie Mischungen und Kombinationen von mindestens zwei der vorgenannten Extrakte;
- Wasser, insbesondere in Mengen von 5 bis 99 Gew.-%, insbesondere 7,5 bis 70 Gew.-%, vorzugsweise 10 bis 50 Gew.-%, besonders bevorzugt 15 bis 40 Gew.-%;
- gegebenenfalls mindestens einen vorzugsweise flüssigen Zucker und/oder Zuckeraustauschstoff, bevorzugt auf Basis einer Mischung von Saccharose und einem Sirup, insbesondere einem Sirup mit Glucose und Fructose, vorzugsweise in etwa gleichem Stoffmengenanteil (Invert-Sirup), insbesondere wobei die Zusammensetzung den mindestens einen Zucker und/oder Zuckeraustauschstoff in Mengen von 5 bis 95 Gew.-%, insbesondere 10 bis 90 Gew.-%, vorzugsweise 20 bis 90 Gew.-%, besonders bevorzugt 40 bis 85 Gew.-%, ganz besonders bevorzugt 60 bis 85 Gew.-%, enthält;
- mindestens ein Additiv aus der Gruppe von Netzmitteln und/oder oberflächenaktiven Substanzen, Konservierungsmitteln, Stabilisatoren, geschmacks- und/oder geruchsmodifizierenden Substanzen, Rheologiemodifikatoren, Verdickungsmitteln, Farbstoffen, Puffern, pH-Stellmitteln, Emulgatoren, Suspensionsmitteln oder deren Mischungen, insbesondere wobei die Zusammensetzung das mindestens eine Additiv in Mengen von 0,001 bis 30 Gew.-%, insbesondere 0,01 bis 20 Gew.-%, vorzugsweise 0,05 bis 10 Gew.-%, besonders bevorzugt 0,1 bis 5 Gew.-%, enthält;
wobei alle vorgenannten Gewichtsprozentangaben auf die Zusammensetzung bezogen sind.

Weiterhin kann die erfindungsgemäße Zusammensetzung, insbesondere pharmazeutische Zubereitung, wie sie zuvor beschrieben wurde, zur prophylaktischen und/oder therapeutischen Behandlung von Atemwegserkrankungen, Erkältungskrankheiten sowie grippalen Infekten und damit verbundenen Symptomen, eingesetzt werden.

Für weitergehende Einzelheiten zu dem zweiten Erfindungsaspekt kann zur Vermeidung unnötiger Wiederholungen auf obige Ausführungen zu dem ersten Erfindungsaspekt verwiesen werden, welche in Bezug auf den zweiten Erfindungsaspekt entsprechend gelten.
Weiterhin ist Gegenstand der vorliegenden Erfindung - gemäß einem **dritten** Aspekt der vorliegenden Erfindung - die Verwendung einer Zusammensetzung nach der vorliegenden Erfindung zur Herstellung eines Arzneimittels zur prophylaktischen und/oder therapeutischen Behandlung von Atemwegserkrankungen, Erkältungskrankheiten sowie grippalen Infekten und damit verbundenen Symptomen.
Für weitergehende Einzelheiten zu diesem Erfindungsaspekt kann zur Vermeidung unnötiger Wiederholungen auf obige Ausführungen zu den übrigen Erfindungsaspekten verwiesen werden, welche in Bezug auf diesen Erfindungsaspekt entsprechend gelten.
Schließlich eignet sich die erfindungsgemäße Zusammensetzung für ein Verfahren zur prophylaktischen und/oder therapeutischen Behandlung von Atemwegserkrankungen, Erkältungskrankheiten sowie grippalen Infekten und damit verbundenen Symptomen, wobei dazu eine erfindungsgemäße Zusammensetzung, wie sie zuvor beschrieben worden ist, in therapeutisch wirksamen Mengen verabreicht wird.

Die vorliegende Erfindung wird anhand der folgenden Ausführungsbeispiele veranschaulicht.

### Ausführungsbeispiele:

### 1. Herstellung der alkoholischen Ausgangsextrakte von Thymian und/oder Primelwurzel

Zunächst wurden separate alkoholische Extrakte von Thymian einerseits und Primelwurzel andererseits hergestellt. Die Herstellung der alkoholischen Extrakte erfolgte in an sich bekannter Weise. Als Auszugsmittel zur Herstellung des alkoholischen Thymianextraktes diente ein Lösemittelgemisch aus 10%iger Ammoniaklösung (1 Gewichtsteil), 85%igem Glycerin (20 Gewichtsteile), 90%igem Ethanol (70 Gewichtsteile) und Wasser (109 Gewichtsteile); es resultierte ein flüssiger alkoholischer Thymianextrakt mit einem Droge/Extrakt-Verhältnis von 1 : 2-2,5.

Zur Herstellung des Primelwurzelextraktes wurde als Auszugsmittel 70%iges Ethanol eingesetzt; es resultierte ein flüssiger alkoholischer Primelwurzelextrakt gleichermaßen mit einem Droge/Extrakt-Verhältnis von 1 : 2-2,5.

Die auf diese Weise hergestellten Extrakte wurden mit einem Gewichtsverhältnis von Thymianflüssigextrakt/Primelwurzelflüssigextrakt von 2 : 1 vereinigt. Nachfolgend wurde ein Chromatogramm dieses Mischextraktes erstellt, um in den nachfolgenden Versuchen festzustellen, ob die Inhalts- bzw. Wirkstoffe der beiden Extrakte bei der nachfolgenden Behandlung erhalten bleiben. Des Weiteren wurde der Thymolgehalt in dem Extraktgemisch bestimmt, da das Thymol nachfolgend als Indikatorsubstanz für den Nachweis einer etwaig auftretenden Veränderung des Extraktes bei der nachfolgenden Behandlung dient; Thymol, ein flüchtiges aromatisches Monoterpen und Bestandteil des Thymianextraktes, eignet sich in diesem Zusammenhang in besonderem Maße als diesbezügliche Indikatorsubstanz für den Thymian-Extrakt. Analog dazu wurde zur Untersuchung des Primelwurzelextrakts der Gehalt an Primulasäure A bestimmt.

### 2. Herstellung alkoholfreier Zusammensetzungen auf Basis von Thymian-und Primelwurzelextrakten und diesbezügliche Vergleichsversuche

Zur Herstellung der zumindest im Wesentlichen alkoholfreien Zusammensetzung auf pflanzlicher Basis wurde zunächst der Alkoholgehalt der bereitgestellten alkoholischen Pflanzenextrakte mittels Membranfiltrationsverfahren reduziert. Für die Vergleichsversuche wurden stets Thymianflüssigextrakt und Primelwurzelflüssigextrakt in einem Verhältnis von 2 : 1 eingesetzt, und zwar unabhängig davon, ob Einzel- oder Mischextrakte filtriert wurden. Zudem wurden Testreihen zur Filtration von Einzel- bzw. Mischextrakten mit verschiedenen Filtrationsanlagen durchgeführt. Auch wurde der Einfluss von Lösungsvermittlern und der Alkoholkonzentration des eingesetzten Extrakts auf die Extraktionseffizienz untersucht, um eine effektive Entfernung des Alkohols einerseits und einen möglichst geringen Substanzverlust der pharmakologisch wertvollen Inhaltsstoffe andererseits zu ermöglichen.

### Versuchsreihe 1: Membranfiltration

### Entalkoholisierung des Primelwurzelextrakts

Zur Herstellung eines alkoholfreien Primelwurzelextrakts wurde der wie im vorherigen Abschnitt beschriebene Primelwurzelextrakt zur Membranfiltration unter Verwendung einer üblichen Labormembranfiltereinheit eingesetzt.

Die Filtrationseffizienz wurde anhand einer Analyse des Permeats und des Retentats auf Ethanol einerseits und Primulasäure A andererseits untersucht. Primulasäure A stellt die anerkannte analytische Markersubstanz zur Messung des Gehalts an Primelwurzelextrakt in pharmazeutischen Zusammensetzungen dar. Im Rahmen der vorherigen Erfindung sollten die Anteile der Primulasäure A im Retentat der Filtration gegenüber dem ursprünglich eingesetzten Primelwurzelextrakt zumindest im Wesentlichen unverändert vorliegen.

Zunächst wurde der Primelwurzelextrakt in unverdünnter Form mit einer Ethanolkonzentration von circa 73 Vol.-% mit Hilfe der vorgenannten Labormembranfiltereinheit filtriert. Die anschließende Analyse von Permeat und Retentat zeigte jedoch, dass bei der Filtration von unverdünntem Primelwurzelextrakt ein gewisser, wenn auch noch akzeptabler Verlust von Primulasäure A auftritt.

In einer weiteren Versuchsreihe unter Einsatz einer mehrstufigen Verdünnungskaskade wurde der Primelwurzelextrakt in verdünnter Form zur Membranfiltration eingesetzt. Die sechsstufige Verdünnungskaskade wurde bei einer Ausgangskonzentration von 25 Vol.-% Ethanol im Primelwurzelextrakt begonnen. Nach sechs Filtrationsschritten wies das Permeat noch ein Ethanol-Gehalt von 0,391 Vol.-% auf.

Im Folgenden sind die Ethanol-Gehalte [Vol.-%] der sechsstufigen Verdünnungskaskade mit den Schritten 1a bis 6a angegeben:

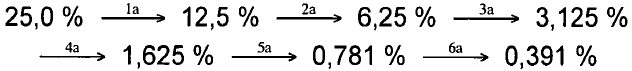

Die Permeate der Verdünnungsstufen 1a bis 6a sowie das resultierende Retentat wurden auf ihren Ethanol- und Primulasäure A-Gehalt untersucht. Die Analyseergebnisse sind in der nachfolgenden Tabelle dargestellt:

| | Primulasäure A-Gehalt | Ethanol-Gehalt [Vol.-%] |
|---|---|---|
| Permeat 1 a | < 0,06 mg/100 ml | 12,500 |
| Permeat 2a | < 0,06 mg/100 ml | 6,250 |
| Permeat 3a | < 0,06 mg/100 ml | 3,125 |
| Permeat 4a | < 0,06 mg/100 ml | 1,625 |
| Permeat 5a | < 0,06 mg/100 ml | 0,781 |
| Permeat 6a | < 0,06 mg/100 ml | 0,391 |
| Retentat a | 98 % Primulasäure A gegenüber dem ursprünglichen Extrakt | 0,391 |
| Detektionsgrenze von Primulasäure A: | | 0,06 mg/100 ml |
| Bestimmungsgrenze der Primulasäure A: | | 0,17 mg/100 ml |

Anhand der Analyseergebnisse wurde deutlich, dass der Ethanolgehalt des ursprünglichen Extrakts von 73 Vol.-% auf 0,391 Vol.-% reduziert werden konnte.

Primulasäure A und andere, insbesondere niedermolekulare, Komponenten konnten die Membran nicht bzw. zumindest nicht in signifikanten Mengen passieren, da ihre Konzentration im Permeat unterhalb der Detektionsgrenze lag. Der Fingerprint des finalen Retentats war vergleichbar mit dem des ursprünglichen Flüssigextrakts.

Der Vergleich des Gehalts an Primulasäure A im ursprünglichen Primelwurzelflüssigextrakts mit dem Gehalt an Primulasäure A im Retentat der Membranfiltration mittels HPLC Fingerprint zeigte, dass die Ausbeute an Primulasäure A im Retentat bei etwa 98 Vol.-% lag.

Zur Untersuchung des Verbleibs der fehlenden 2 Vol.-% an Primulasäure A im Retentat wurde die verwendete Membran einer Extraktion unterzogen. Anschließend wurden sowohl das Extraktionsmedium als auch die Filtrationsreste untersucht. Dabei konnten 82,7 % der im Filtrationsretentat fehlenden Primulasäure A detektiert werden. Insgesamt betrug die Ausbeute an Primulasäure A somit mehr als 99 %.

Insgesamt kann zusammengefasst werden, dass der Ethanolgehalt im Primelwurzelextrakt von 73 Vol.-% auf 0,391 Vol.-% bei einer Wirkstoffausbeute von 99 % im Retentat reduziert werden konnte.

Anhand der Resultate wird deutlich, dass es möglich ist, ohne einen nennenswerten Substanzverlust der pharmakologisch wertvollen Inhaltstoffe den Ethanol-Anteil aus Primelwurzelextrakt mittels Membranfiltration effizient zu entfernen.

### Entalkoholisierung des Thymianflüssigextrakts

Zur Herstellung eines alkoholfreien Thymianflüssigextrakts wurde ebenfalls der wie im vorherigen Abschnitt beschrieben hergestellte Thymianflüssigextrakt zur Membranfiltration unter Verwendung der vorgenannten Labormembranfiltereinheit eingesetzt.

Die Filtrationseffizienz wurde anhand einer Analyse des Permeats und des Retentats auf das Vorliegen von Ethanol einerseits und Thymol andererseits untersucht. Thymol stellt die anerkannte analytische Markersubstanz zur Messung des Gehalts an Thymianextrakt in pharmazeutischen Zusammensetzungen dar. Im Rahmen der vorliegenden Erfindung sollten die Anteile des Thymols im Retentat der Filtration gegenüber dem ursprünglich eingesetzten Thymianextrakt zumindest im Wesentlichen unverändert vorliegen.

Zunächst wurde der Thymianflüssigextrakt in unverdünnter Form bei einer Ethanolausgangskonzentration von circa 32 Vol.-% zur Membranfiltration mit Hilfe der vorgenannten Labormembranfiltereinheit eingesetzt. Die anschließende Analyse von Permeat und Retentat zeigte jedoch, dass bei der Membranfiltration von unverdünntem Thymianextrakt bzw. Thymianextrakt ein gewisser, wenn auch noch akzeptabler Verlust von Thymol auftritt.

In einer weiteren Versuchsreihe unter Einsatz einer mehrstufigen Verdünnungskaskade wurde der Thymianextrakt in verdünnter Form eingesetzt. Die fünfstufige Verdünnungskaskade wurde bei einer Ausgangskonzentration von 15 Vol.-% Ethanol im Thymianextrakt begonnen. Nach fünf Filtrationsschritten wies das Permeat noch eine Ethanol-Konzentration von 0,469 Vol.-% auf.

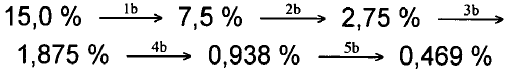

Die Permeate der Verdünnungsstufen 1 b bis 5b sowie das resultierende Retentat wurden auf ihre Ethanol- und Thymol-Konzentration untersucht. Die Analyseergebnisse sind in der nachfolgenden Tabelle dargestellt:

| | Thymol-Gehalt | Ethanol-Gehalt [Vol.-%] |
|---|---|---|
| Permeat 1 b | < 0,003 mg/100 ml | 7,500 |
| Permeat 2b | < 0,003 mg/100 ml | 3,750 |
| Permeat 3b | < 0,003 mg/100 ml | 1,875 |
| Permeat 4b | < 0,003 mg/100 ml | 0,938 |
| Permeat 5b | < 0,003 mg/100 ml | 0,469 |
| Retentat b | 97,5 % Thymol gegenüber dem ursprünglichen Extrakt | 0,469 |
| Detektionsgrenze von Thymol: | | 0,003 mg/100 ml |
| Bestimmungsgrenze von Thymol: | | 0,009 mg/100 ml |

Die Resultate zeigen, dass der Ethanolgehalt des ursprünglich eingesetzten Thymianextrakts von 32 Vol.-% auf 0,469 Vol.-% reduziert werden konnte. Thymol sowie weitere niedermolekulare Komponenten konnten die Membran nicht bzw. zumindest nicht in signifikanten Mengen passieren, da ihr Gehalt im Permeat unterhalb der Nachweisgrenze lag. Der Fingerprint des finalen Retentats war vergleichbar mit dem des ursprünglich eingesetzten Thymianextrakts.

Der Vergleich des Thymolgehalts des ursprünglich eingesetzten Thymianextrakts mit dem des Retentats der Membranfiltration mittels Gaschromatographie zeigte, dass die Ausbeute von Thymol im Retentat 97,5 % betrug.

Zur Untersuchung des Verbleibs der fehlenden 2,5 Vol.-% an Thymol im Retentat wurde die verwendete Membran einer Extraktion unterzogen. Anschließend wurden sowohl das Extraktionsmedium als auch die Filtrationsreste untersucht. Dabei konnten mehr als 95 % des im Filtrationsretentat fehlenden Thymols detektiert werden. Ingesamt betrug die Ausbeute an Thymol somit mehr als 99 %.

### Entalkoholisierung eines Thymian/Primelwurzel-Mischextrakts

Um den Prozess hinsichtlich einer Zeiteinsparung zu verbessern, wurde die Membranfiltration mittels der vorgenannten Labormembranfiltrationseinheit mit einem Thymian/Primelwurzel-Mischextrakt durchgeführt.

Dazu wurden die gemäß dem vorangehenden Abschnitt hergestellten Extrakte auf Basis von Thymian bzw. Primelwurzeln im Verhältnis von 2 : 1 gemischt. Weiterhin wurden dem Gemisch zur Vermeidung der Bildung von Präzipitaten 10 Gew.-% Poly(oxyethylen)-40-hydriertes Rizinusöl als Lösungsvermittler (Netz- bzw. Dispergiermittel) hinzugefügt. Die Mischung hatte somit einen Gesamtethanolgehalt von 45,7 Vol.-%.

Die Filtration wurde in einer fünfstufigen Verdünnungskaskade durchgeführt, beginnend bei einem Ethanolgehalt von 15 Vol.-%. Nach den fünf Filtrationsschritten wies das Permeat noch einen Ethanolgehalt von 0,469 Vol.-% auf.

Im Folgenden sind die Ethanolgehalte [Vol.-%] der fünfstufigen Verdünnungskaskade mit den Schritten 1 c bis 5c angegeben:

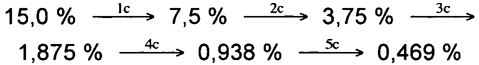

Die Permeate der Verdünnungsstufen 1 c bis 5c sowie das resultierende Retentat wurden auf ihren Gehalt an Ethanol, Thymol sowie Primulasäure A untersucht. Die Analyseergebnisse sind in der nachfolgenden Tabelle dargestellt:

| | Thymol-Gehalt | Primulasäure A-Gehalt | | Ethanol-Gehalt [Vol.-%] |
|---|---|---|---|---|
| Permeat 1c | < 0,003 mg/100 ml | < 0,06 mg/100 ml | | 7,500 |
| Permeat 2c | < 0,003 mg/100 ml | < 0,06 mg/100 ml | | 3,750 |
| Permeat 3c | < 0,003 mg/100 ml | < 0,06 mg/100 ml | | 1,875 |
| Permeat 4c | < 0,003 mg/100 ml | < 0,06 mg/100 ml | | 0,938 |
| Permeat 5c | < 0,003 mg/100 ml | < 0,06 mg/100 ml | | 0,469 |
| Retentat c | ca. 99 % Thymol gegenüber dem ursprünglichen Mischextrakt | ca. 98 % Primulasäure A gegenüber dem ursprünglichen Mischextrakt | | 0,469 |
| Detektionsgrenze von Primulasäure A: | | | 0,06 mg/100 ml | |
| Bestimmungsgrenze der Primulasäure A: | | | 0,17 mg/100 ml | |
| Detektionsgrenze von Thymol: | | | 0,003 mg/100 ml | |
| Bestimmungsgrenze von Thymol: | | | 0,009 mg/100 ml | |

Die Resultate zeigen, dass der Ethanolgehalt im ursprünglichen Mischextrakt von 45,7 Vol.-% auf 0,469 Vol.-% im Retentat der Membranfiltration reduziert werden konnte. Primulasäure A, Thymol sowie weitere niedermolekulare Komponenten bzw. pharmakologisch relevante Inhalts- bzw. Wirkstoffe konnten die Membran nicht bzw. zumindest nicht in signifikanten Mengen passieren. Sowohl die Konzentration von Thymol als auch die von Primulasäure A lagen unterhalb der Detektion- bzw. Bestimmungsgrenze.

Zur Bestimmung der Ausbeute von Thymol bzw. Primulasäure A wurde jeweils vom ursprünglich eingesetzten Mischextrakt sowie vom Retentat der Filtration ein HPLC-Fingerprint angefertigt. Dabei zeigte sich für Primulasäure A eine Ausbeute von etwa 98 %. Die Ausbeute von Thymol betrug etwa 99 %.

Insgesamt konnte somit auch aus einem Thymian/Primelwurzel-Mischextrakt der Alkohol bei gleichzeitig guter Ausbeute der Markersubstanzen Thymol und Primulasäure A effizient entfernt werden.

### Versuchsreihe 2: Membranfiltration

In einer zweiten Versuchsreihe wurde eine andere Membranfiltrationsvorrichtung mit einer anderen Membran verwendet, welche auch zur Filtration unverdünnter Extrakte in einem einzelnen Filtrationsschritt geeignet ist. Dabei wurde die Filtrationseffizienz sowohl von Einzel- als auch von Mischextrakten analysiert.

### Entalkoholisierung des Primelwurzelextrakts

Zur Herstellung eines alkoholfreien Primelwurzelextrakts in einem einzelnen Membranfiltrationsschritt wurde der gemäß dem vorigen Abschnitt hergestellte Primelwurzelextrakt in unverdünnter Form eingesetzt. Der Alkoholgehalt im Ausgangsextrakt betrug dabei 73 Vol.-%. Nach erfolgter Filtration betrug der Ethanolgehalt circa 0,387 Vol.-%. Die Ausbeute an Primulasäure A im Retentat betrug circa 98,5 %.

### Entalkoholisierung des Thymianextrakts

Zur Herstellung eines alkoholfreien Thymianextrakts in einem einzelnen Membranfiltrationsschritt wurde der gemäß dem vorigen Abschnitt hergestellte Thymianextrakt in unverdünnter Form eingesetzt. Der Alkoholgehalt im Ausgangsextrakt betrug dabei circa 30 Vol.-%. Nach erfolgter Filtration betrug der Ethanolgehalt circa 0,421 Vol.-%. Die Ausbeute an Thymol im Retentat betrug circa 99,2 %.

### Filtration eines Thymian/Primelwurzel-Mischextrakts

Die gemäß dem vorigen Abschnitt hergestellten Extrakte auf Basis von Thymian bzw. Primelwurzeln wurden im Verhältnis von 2 : 1 gemischt. Weiterhin wurden dem Gemisch zur Vermeidung der Bildung von Präzipitaten 10 Gew.-% Poly(oxyethylen)-40-hydriertes Rizinusöl als Lösungsvermittler (Netz- bzw. Dispergiermittel) hinzugefügt. Die Mischung hatte einen Gesamtethanolgehalt von 45,7 Vol.-%. Nach der Filtration wurden Permeat und Retentat der Filtration auf ihren Gehalt an Ethanol, Thymol und Primulasäure A untersucht. Die nachfolgende Tabelle zeigt die Analyseergebnisse für den Mischextrakt:

| | Thymol-Konzentration | Primulasäure A-Konzentration | | Ethanol-Konzentration [Vol.-%] |
|---|---|---|---|---|
| Permeat d | < 0,06 mg/100 ml | 0,003 mg/100 ml | | 0,395 |
| Retentat d | ca. 98,5 % Thymol gegenüber dem ursprünglichen Extrakt | ca. 99,5 % Primulasäure A gegenüber dem ursprünglichen Extrakt | | 0,395 |
| Detektions grenze von Primulasäure A: | | | 0,06 mg/100 ml | |
| Bestimmun gsgrenze der Primulasäure A: | | | 0,17 mg/100 ml | |
| Detektions grenze von Thymol: | | | 0,003 mg/100 ml | |
| Bestimmun gsgrenze von Thymol: | | | 0,009 mg/100 ml | |

### 3. Herstellung einer erfindungsgemäßen Zusammensetzung

Auf Basis der gemäß dem vorigen Abschnitt hergestellten alkoholfreien Pflanzenextrakte aus der 1. Versuchsreihe wurden zwei Beispiele einer erfindungsgemäßen Zusammensetzung hergestellt. Die Probe Nr. 1 wurde auf Basis der separat filtrierten Retentate hergestellt, während die Probe Nr. 2 auf Basis des aus dem Thymian/Primelwurzelmisch-Extrakt resultierenden Retentats hergestellt wurde.

Der folgenden Tabelle können die eingesetzten Mengen und Funktionen der jeweiligen Inhaltsstoffe entnommen werden:

| Rohmaterialien | Funktion | Probe Nr. 1 [pro 100 g] | Probe Nr. 2 [per 100 g] |
|---|---|---|---|
| Alkoholfreier Thymianflüssigextrakt (DEV = 1 : 2-2,5) | Arzneidroge | 5,00 | --- |
| alkoholfreier Primelwurzelflüssigextrakt (DEV = 1 : 2-2,5) | Arzneidroge | 2,50 | --- |
| alkoholfreier Thymian/PrimelwurzelMischextrakt (2:1) (DEV = 1 : 2-2,5) | Arzneidroge | --- | 7,50 |
| Natriumbenzoat | Konservierungsmittel | 0,18 | 0,18 |
| Dispergiermittel | Lösungsvermittler | --- | 5,00 |
| Gemisch aus Zucker und Invertsirup | Geschmack, Lösungsmittel | 85,00 | 80,00 |
| Purified water | Lösungsmittel | 7,32 | 7,32 |

Der Ethanolgehalt der Probe Nr. 1 betrug 1.600 ppm. Die Probe Nr. 2 wies einen Rest-Ethanolgehalt von 2.800 ppm auf. Beide erfindungsgemäß hergestellten Zusammensetzungen wiesen somit einen Ethanolgehalt von weniger als 5.000 ppm auf und sind somit im Sinne der deutschen Arzneimittelwarenverordnung bzw. des Europäischen Arzneibuchs als alkoholfrei anzusehen.

Die dünnschichtchromatographischen Fingerprints zeigen, dass das alkoholfreie Arzneimittelprodukts mit dem alkoholbasierten Produkt vergleichbar ist und auch mit dem Fingerprint des Originalextrakts übereinstimmt. Die anerkannten analytischen Markersubstanzen (Thymol, Primulasäure A) konnten neben den übrigen Komponenten des Originalextrakts mittels Dünnschichtchromatographie (TLC) detektiert werden.

Die Analyseergebnisse zeigen, dass es möglich ist, einen alkoholfreien Extrakt unter Durchführung eines Membranfiltrationsprozesses herzustellen. Dabei kann Ethanol erfolgreich aus beiden vorgenannten flüssigen Pflanzenextrakten entfernt werden. Die anerkannten analytischen Markersubstanzen des Endproduktes (Thymol und Primulasäure A) sowie weitere, insbesondere niedermolekulare Komponenten, können die Membran nicht passieren, insbesondere dann nicht, wenn die Flüssigextrakte in verdünnter Form gefiltert werden. Dennoch liegt die absolute Ausbeute der Markersubstanz Thymol unter 95 %. Um eine etwaige Präzipitation im Rahmen des Filtrationsprozesses zu vermeiden, kann gegebenenfalls zusätzlich ein Lösungsvermittler (der Lebensmittelnorm entsprechend) verwendet werden, wobei diesbezüglich die hierfür dem Fachmann an sich bekannten Netz- und Dispergiermittel eingesetzt werden können.

Im Ergebnis stellt das erfindungsgemäße Verfahren eine effiziente wie kostengünstige Methode dar, um zumindest im Wesentlichen alkoholfreie Zusammensetzungen auf Basis von Thymian- und/oder Primelwurzelextrakten, aber auch anderer Pflanzenextrakte mit sekretolytischer bzw. expektorationsfördernder Wirkung, zu erhalten.

In analoger Vorgehensweise lassen sich nämlich auch erfindungsgemäße Zusammensetzungen auf Basis anderer Pflanzenextrakte der vorgenannten Art (z. B. Efeu-Extrakt, Holunder-Extrakt, Fenchel-Extrakt, Huflattich-Extrakt, Anis-Extrakt und Tüpfelfarn-Extrakt sowie Mischungen und Kombinationen von mindestens zwei der vorgenannten Extrakte) herstellen.

### 4. Vergleichsversuche

Nachfolgend werden nicht erfindungsgemäße Vergleichsversuche beschrieben:
In einem ersten, jedoch nicht erfindungsgemäßen Ansatz wird der wie zuvor beschrieben hergestellte Mischextrakt von Thymian und Primelwurzel einer Vakuumtrocknung unterzogen, um das Extraktionsmittel, einschließlich des Alkohols, zu entfernen. Der resultierende Rückstand wird in Wasser aufgenommen und chromatographisch untersucht. Es zeigt sich, dass nicht nur ein deutlicher Substanzverlust (> 50 %) eingetreten ist, sondern darüber hinaus charakteristische, insbesondere flüchtige Inhaltsstoffe der beiden Extrakte bei der auf diese Weise erfolgten Behandlung verloren gegangen sind. Über eine Vakuumtrocknung lässt sich ein nichtalkoholischer Extrakt bzw. eine nichtalkoholische Zusammensetzung auf Basis der beiden vorgenannten Extrakte folglich nicht erhalten. Vergleichbare Ergebnisse erhält man, wenn man andere Trocknungsmethoden anwendet, insbesondere Gefriertrocknung oder Trocknung unter erhöhten Temperaturen. Reflektiert an dem repräsentativen Inhaltsstoff Thymol aus dem Thymianextrakt, werden nach den Trocknungsversuchen jeweils nur 6,4 % bis 16,78 % des ursprünglich vorliegenden Thymols im resultierenden getrockneten Extrakt erhalten; das restliche Thymol wird im Rahmen der Trocknung entfernt.

Mit einfachen Evaporationsverfahren lässt sich der Ethanolgehalt nicht ohne Weiteres reduzieren.

### 5. Anwendungsbeobachtungen

Eine erfindungsgemäße, wie zuvor beschrieben hergestellte alkoholfreie Zusammensetzung auf Basis von Thymian- und Primelwurzelextrakten eignet sich insbesondere zur Verabreichung bei Säuglingen und Kleinkindern.

19 Probanden im Alter zwischen 6 und 33 Monaten (9 weiblich, 10 männlich), welche an Erkältungskrankheiten der oberen und unteren Atemwege mit zähflüssigem Schleim und Husten erkrankt waren, wurden mit der erfindungsgemäßen Zusammensetzung behandelt. Bei den Probanden im Alter von 6 bis 12 Monaten wurde sechsmal täglich 1,0 ml der erfindungsgemäßen Zusammensetzung über den Tag verteilt verabreicht, während bei den Probanden im Alter von mehr als 12 Monaten eine sechsmalige Verabreichung von Dosen à 2,5 ml erfolgte.

Bereits nach drei- bis fünfmaliger Gabe der Einzeldosen trat in allen Fällen eine erhebliche Verbesserung des Allgemeinzustands auf. Insbesondere wurden ein erleichtertes Abhusten und eine verbesserte Verflüssigung des Schleims beobachtet.

Bei 16 der 19 Probanden war die Therapie nach 5 Tagen abgeschlossen, während bei den übrigen Probanden die Therapie noch weitere 6 Tage fortgeführt wurde, bis sie beendet werden konnte. In allen Fällen waren die Erkrankungen nach dieser Zeitdauer austherapiert.

Bei 8 der 19 Probanden wurden zusätzlich zu der erfindungsgemäßen Zusammensetzung ergänzende Therapeutika, insbesondere Rhinologika, verabreicht.

Weiterhin wurden 45 erwachsene Probanden im Alter von 18 bis 81 Jahren (16 männlich, 29 weiblich), welche an Erkältungskrankheiten der oberen und unteren Atemwege, verbunden mit Husten, zähflüssigem Schleim und zum Teil akuter Bronchitis, litten, mit der erfindungsgemäßen Zusammensetzung-entweder unterstützend oder als Monotherapie - behandelt. Zu diesem Zweck wurden 4- bis 6mal täglich 7,5 bis 10 ml der erfindungsgemäßen Zusammensetzung verabreicht. In allen Fällen wurde bereits nach 1 bis 2 Tagen eine erhebliche Linderung der Erkältungssymptome beobachtet. Nach 9 bis 12 Tagen waren in allen Fällen die Erkrankungen austherapiert.

Die vorstehenden Anwendungsbeobachtungen zeigen die Wirksamkeit der erfindungsgemäßen Zusammensetzung bei der Behandlung von Erkältungskrankheiten der Atemwege und den damit verbundenen Symptomen, wie Husten, zähflüssigem Schleim etc. Aufgrund der Tatsache, dass die erfindungsgemäße Zusammensetzung alkoholfrei ausgebildet ist, eignet sie sich insbesondere auch zum Einsatz bei Säuglingen und Kleinkindern. Des Weiteren wurden die organoleptischen Eigenschaften der alkoholfreien, im vorliegenden Fall konkret Thymian- und Primelwurzelextrakt enthaltenden Zusammensetzung von allen Probanden als äußerst positiv bewertet.

## Patentansprüche

1. Verfahren zur Herstellung einer zumindest im Wesentlichen alkoholfreien Zusammensetzung auf Basis mindestens eines Pflanzenextraktes mit sekretolytischer und/oder expektorationsfördernder Wirkung, wobei die Zusammensetzung einen Alkoholgehalt, insbesondere einen Ethanolgehalt, von weniger als 2 Vol.-% aufweist,
wobei das Verfahren die folgenden Verfahrensschritte umfasst:
(a) Bereitstellung mindestens eines alkoholischen, insbesondere ethanolischen Pflanzenextraktes mit sekretolytischer und/oder expektorationsfördernder Wirkung mit einem Droge/Extrakt-Verhältnis im Bereich von 1 : 10 bis 10 : 1, wobei der mindestens eine Pflanzenextrakt mit sekretolytischer und/oder expektorationsfördernder Wirkung ausgewählt ist aus der Gruppe von Thymian-Extrakt (*Thymus vulgaris*), Primelwurzel-Extrakt *(Primula),* Efeu-Extrakt (*Hedera helix*), Holunder-Extrakt (*Sambucus nigra*), Fenchel-Extrakt (*Foeniculum vulgare*), Huflattich-Extrakt (*Tussilago farfara*), Anis-Extrakt (*Pimpinella anisum*) und Tüpfelfarn-Extrakt (*Polypodium vulgare*) sowie Mischungen und Kombinationen von mindestens zwei der vorgenannten Extrakte; und
(b) nachfolgend Entfernen des Alkohols, insbesondere des Ethanols, mittels Membranfiltration zum Erhalt einer im Wesentlichen alkoholfreien Zusammensetzung mit einem Alkoholgehalt, insbesondere einem Ethanolgehalt, von weniger als 2 Vol.-%, auf Basis mindestens eines Pflanzenextraktes mit sekretolytischer und/oder expektorationsfördernder Wirkung, wobei vor der Durchführung der Membranfiltration mindestens ein Dispergier- oder Netzmittel, vorzugsweise ein Lösungsvermittler, zugesetzt wird und/oder wobei die Membranfiltration in Gegenwart mindestens eines Dispergier- oder Netzmittels, vorzugsweise Lösungsvermittlers, durchgeführt wird, wobei das Dispergier- oder Netzmittel ausgewählt ist aus der Gruppe von organischen Ölen, Fetten und Fettsäuren, Lecithinen, Glyceriden sowie deren Kombinationen und wobei das Dispergier- oder Netzmittel in Mengen von 0,1 bis 30 Gew.-% eingesetzt wird, bezogen auf die der Membranfiltration zu unterziehende Gesamtmischung aus alkoholischem Pflanzenextrakt, Additiv, Wasser und gegebenenfalls weiteren Inhaltsstoffen.

2. Verfahren nach Anspruch 1, **dadurch gekennzeichnet,**
**dass** in Verfahrensschritt (a) der mindestens eine alkoholische Pflanzenextrakt mit einem Droge/Extrakt-Verhältnis im Bereich von 5 : 1 bis 1 : 5, vorzugsweise im Bereich von 3 : 1 bis 1 : 4, bevorzugt im Bereich von 2 : 1 bis 1 : 3,5, ganz besonders bevorzugt im Bereich von 1 : 1 bis 1 : 3, bereitgestellt wird; und/oder
**dass** der in Verfahrensschritt (a) bereitgestellte alkoholische Pflanzenextrakt ein ethanolbasierter Pflanzenextrakt ist; und/oder
**dass** in Verfahrensschritt (a) der mindestens eine alkoholische Pflanzenextrakt mit einem Gehalt an Alkohol, insbesondere Ethanol, von mehr als 10 Vol.-% und/oder mehr als 10 Gew.-%, insbesondere mehr als 20 Vol.-% und/oder mehr als 20 Gew.-%, besonders bevorzugt mehr als 30 Vol.-% und/oder mehr als 30 Gew.-%, bereitgestellt wird und/oder dass in Verfahrensschritt (a) der mindestens eine alkoholische Pflanzenextrakt mit einem Gehalt an Alkohol, insbesondere Ethanol, im Bereich von 10 Vol.-% bis 99 Vol.-% und/oder im Bereich von 10 Gew.-% bis 99 Gew.-%, insbesondere im Bereich von 20 Vol.-% bis 95 Vol.-% und/oder im Bereich von 20 Gew.-% bis 95 Gew.-%, besonders bevorzugt im Bereich von 30 Vol.-% bis 90 Vol.-% und/oder im Bereich von 30 Gew.-% bis 90 Gew.-%, bereitgestellt wird.

3. Verfahren nach einem der vorangehenden Ansprüche, **dadurch gekennzeichnet,**
**dass** in Verfahrensschritt (b) die Membranfiltration als Ultrafiltration, Nanofiltration, Umkehrosmose (Hyperfiltration) oder Dialyse, vorzugsweise als Umkehrosmose (Hyperfiltration) oder Dialyse, bevorzugt als Umkehrosmose (Hyperfiltration), durchgeführt wird; und/oder
**dass** die Membranfiltration, insbesondere Umkehrosmose (Hyperfiltration), mit einer Druckdifferenz und/oder einem Druckgradienten und/oder hydrostatische Druckdifferenz im Bereich von 10 bis 100 bar, insbesondere 20 bis 90 bar, vorzugsweise 30 bis 80 bar, durchgeführt wird.

4. Verfahren nach einem der vorangehenden Ansprüche, **dadurch gekennzeichnet, dass** in Verfahrensschritt (b) die Membranfiltration mittels mindestens eines Membranfilters durchgeführt wird, insbesondere wobei
• das Membranfilter aus anorganischen oder organischen Materialien, insbesondere organischen Materialien, bevorzugt polymeren organischen Materialien, ausgebildet ist, insbesondere wobei das Membranmaterial ausgewählt ist aus der Gruppe von (i) fluorierten und perfluorierten organischen Polymeren, insbesondere fluorierten und perfluorierten Olefinen, vorzugsweise Polytetrafluorethylenen; (ii) Materialien auf Basis von Cellulose oder Cellulosederivaten, insbesondere Celluloseacetaten und regenerierter Cellulose; (iii) sulfonierten organischen Polymeren, insbesondere Polysulfonen und Polyethersulfonen; (iv) Polyolefinen, insbesondere Polyethylenen und Polypropylenen; (v) Polyamiden; (vi) Polyestern sowie deren Kombinationen; und/oder
• das Membranfilter einschichtig oder mehrschichtig, insbesondere als Kompositmaterial, ausgebildet ist; und/oder
• das Membranfilter faserverstärkt ausgebildet ist; und/oder
• das Membranfilter eine Porengröße von höchstens 0,001 µm, insbesondere im Bereich von 0,0001 bis 0,001 µm, aufweist; und/oder
• das Membranfilter eine selektive Permeabilität und/oder Durchlässigkeit aufweist, insbesondere selektiv durchlässig in Bezug auf Alkohol, insbesondere Ethanol, ausgebildet ist und/oder dass das Membranfilter durchlässig, vorzugsweise selektiv durchlässig, in Bezug auf Alkohol, insbesondere Ethanol, ausgebildet ist; und/oder
• das Membranfilter undurchlässig in Bezug auf Wasser ausgebildet ist.

5. Verfahren nach einem der vorangehenden Ansprüche, **dadurch gekennzeichnet,**
**dass** in Verfahrensschritt (b) die Membranfiltration bei Temperaturen im Bereich von 5 bis 80 °C, insbesondere 10 bis 70 °C, vorzugsweise 15 bis 60 °C, besonders bevorzugt 20 bis 50 °C, durchgeführt wird; und/oder
**dass** in Verfahrensschritt (b) die Membranfiltration kontinuierlich oder diskontinuierlich, vorzugsweise kontinuierlich, durchgeführt wird; und/oder
**dass** in Verfahrensschritt (b) die Membranfiltration in einem offenen oder geschlossenen System, vorzugsweise in einem geschlossenen System, durchgeführt wird; und/oder
**dass** dem in Verfahrensschritt (a) bereitgestellten alkoholischen, insbesondere ethanolischen Pflanzenextrakt vor der Durchführung der Membranfiltration Wasser zugesetzt wird, insbesondere wobei der Alkoholanteil des Pflanzenextraktes auf einen Wert von höchstens 40 Vol.-% und/oder 40 Gew.-%, insbesondere höchstens 35 Vol.-% und/oder 35 Gew.-%, vorzugsweise höchstens 30 Vol.-% und/oder 30 Gew.-%, eingestellt wird; und/oder
**dass** das mindestens eine Dispergier- oder Netzmittel in Mengen von 0,5 bis 20 Gew.-%, vorzugsweise 1 bis 15 Gew.-%, eingesetzt wird, bezogen auf die der Membranfiltration zu unterziehende Gesamtmischung aus alkoholischem Pflanzenextrakt, Additiv, Wasser und gegebenenfalls weiteren Inhaltsstoffen.

6. Verfahren nach einem der vorangehenden Ansprüche, **dadurch gekennzeichnet,**
**dass** in Verfahrensschritt (b) die Membranfiltration einstufig oder mehrstufig, vorzugsweise mehrstufig, durchgeführt wird; und/oder
**dass** in Verfahrensschritt (b) die Membranfiltration mehrstufig, vorzugsweise in mindestens zwei, insbesondere mindestens drei aufeinanderfolgenden Stufen (Teilschritten), besonders bevorzugt in drei bis zehn aufeinanderfolgenden Stufen (Teilschritten), durchgeführt wird, insbesondere wobei die Membranfiltration in jeder Stufe derart durchgeführt wird, dass der Alkoholgehalt, insbesondere Ethanolgehalt, bei jeder Stufe um mindestens 20 %, insbesondere um mindestens 30 %, vorzugsweise um mindestens 40 %, bezogen auf den Ausgangsalkoholgehalt zu Beginn der jeweiligen Stufe, reduziert wird und/oder insbesondere wobei zu Beginn jeder Stufe der betreffenden Zusammensetzung vor der Durchführung der Membranfiltration Wasser zugesetzt wird; und/oder
**dass** die in Verfahrensschritt (b) resultierende, im Wesentlichen alkoholfreie Zusammensetzung auf Basis mindestens eines Pflanzenextraktes mit sekretolytischer und/oder expektorationsfördernder Wirkung als Retentat der Membranfiltration erhalten wird.

7. Verfahren nach einem der vorangehenden Ansprüche, **dadurch gekennzeichnet,**
**dass** sich Verfahrensschritt (b) ein Verfahrensschritt (c) anschließt, bei dem die in Verfahrensschritt (b) resultierende, im Wesentlichen alkoholfreie Zusammensetzung auf Basis mindestens eines Pflanzenextraktes mit sekretolytischer und/oder expektorationsfördernder Wirkung auf eine gewünschte finale Zusammensetzung, insbesondere in einer für die perorale Applikation hergerichteten Form, eingestellt wird;
• insbesondere wobei in Verfahrensschritt (c) der in Verfahrensschritt (b) resultierenden, im Wesentlichen alkoholfreien Zusammensetzung auf Basis mindestens eines Pflanzenextraktes mit sekretolytischer und/oder expektorationsfördernder Wirkung Wasser und/oder ein wässriger Exzipient und/oder gegebenenfalls mindestens ein weiterer Inhaltsstoff zugesetzt wird bzw. werden;
• insbesondere wobei der in Verfahrensschritt (c) eingesetzte wässrige Exzipient neben Wasser mindestens ein(en) vorzugsweise flüssigen Zucker, Zuckeraustauschstoff und/oder Kohlenhydrat, bevorzugt auf Basis einer Mischung von Saccharose und einem Sirup, insbesondere einem Sirup mit Glucose und Fructose, vorzugsweise in etwa gleichem Stoffmengenanteil (Invert-Sirup), enthält und/oder dass der in Verfahrensschritt (c) eingesetzte wässrige Exzipient neben Wasser mindestens ein weiteres Additiv, insbesondere aus der Gruppe von Dispergier- oder Netzmitteln und/oder oberflächenaktiven Substanzen, Konservierungsmitteln, Stabilisatoren, geschmacks- und/oder geruchsmodifizierenden Substanzen, Rheologiemodifikatoren, Verdickungsmitteln, Farbstoffen, Puffern, pH-Stellmitteln, Emulgatoren, Suspensionsmitteln oder deren Mischungen, enthält und/oder dass der in Verfahrensschritt (c) eingesetzte wässrige Exzipient Wasser in Mengen von 5 bis 95 Gew.-%, insbesondere 10 bis 90 Gew.-%, vorzugsweise 20 bis 80 Gew.-%, bezogen auf den Exzipienten, enthält; und/oder
• insbesondere wobei der in Verfahrensschritt (c) eingesetzte weitere Inhaltsstoff ausgewählt ist aus der Gruppe von vorzugsweise flüssigen Zuckern, Zuckeraustauschstoffen und/oder Kohlenhydraten, bevorzugt auf Basis einer Mischung von Saccharose und einem Sirup, insbesondere einem Sirup mit Glucose und Fructose, vorzugsweise in etwa gleichem Stoffmengenanteil (Invert-Sirup), und/oder Additiven, insbesondere Netzmitteln und/oder oberflächenaktiven Substanzen, Konservierungsmitteln, Stabilisatoren, geschmacks- und/oder geruchsmodifizierenden Substanzen, Rheologiemodifikatoren, Verdickungsmitteln, Farbstoffen, Puffern, pH-Stellmitteln, Emulgatoren, Suspensionsmitteln oder deren Mischungen.

8. Verfahren nach einem der vorangehenden Ansprüche, **dadurch gekennzeichnet,**
**dass** die Zusammensetzung in Verfahrensschritt (b) und/oder (c) auf einen Alkoholgehalt, insbesondere einen Ethanolgehalt, von weniger als 1,0 Vol.-%, vorzugsweise weniger als 0,85 Vol.-%, besonders bevorzugt weniger als 0,5 Vol.-%, ganz besonders bevorzugt weniger als 0,25 Vol.-%, noch mehr bevorzugt weniger als 0,1 Vol.-%, am meisten bevorzugt auf etwa 0 Vol.-%, eingestellt wird; und/oder
**dass** die Zusammensetzung auf eine unter Normalbedingungen, insbesondere bei einer Temperatur von 25 °C und unter einem Druck von 1,01325 bar, flüssige und/oder fließfähige Viskosität oder Konsistenz eingestellt wird.

9. Zusammensetzung auf Basis mindestens eines Pflanzenextraktes mit sekretolytischer und/oder expektorationsfördernder Wirkung, insbesondere pharmazeutische Zubereitung, vorzugsweise zur Verwendung zur prophylaktischen und/oder therapeutischen Behandlung von Atemwegserkrankungen, Erkältungskrankheiten sowie grippalen Infekten und damit verbundenen Symptomen, erhältlich durch ein Verfahren nach einem der vorangehenden Ansprüche.

10. Zusammensetzung, insbesondere pharmazeutische Zubereitung, vorzugsweise zur Verwendung zur prophylaktischen und/oder therapeutischen Behandlung von Atemwegserkrankungen, Erkältungskrankheiten sowie grippalen Infekten und damit verbundenen Symptomen, erhältlich durch ein Verfahren nach einem der vorangehenden Ansprüche, wobei die Zusammensetzung in Form einer zumindest im Wesentlichen alkoholfreien Zubereitung vorliegt, wobei die Zusammensetzung einen Alkoholgehalt, insbesondere einen Ethanolgehalt, von weniger als 2 Vol.-%, aufweist, wobei die Zusammensetzung mindestens ein Dispergier- oder Netzmittel, vorzugsweise Lösungsvermittler, enthält, wobei das Dispergier- oder Netzmittel ausgewählt ist aus der Gruppe von organischen Ölen, Fetten und Fettsäuren, Lecithinen, Glyceriden sowie deren Kombinationen und wobei der mindestens eine Pflanzenextrakt mit sekretolytischer und/oder expektorationsfördernder Wirkung ausgewählt ist aus der Gruppe von Thymian-Extrakt (*Thymus vulgaris*), Primelwurzel-Extrakt (*Primula*), Efeu-Extrakt (*Hedera helix*), Holunder-Extrakt (*Sambucus nigra*), Fenchel-Extrakt (*Foeniculum vulgare*), Huflattich-Extrakt (*Tussilago farfara*), Anis-Extrakt (*Pimpinella anisum*) und Tüpfelfarn-Extrakt (*Polypodium vulgare*) sowie Mischungen und Kombinationen von mindestens zwei der vorgenannten Extrakte, wobei der Pflanzenextrakt ein Droge/Extrakt-Verhältnis im Bereich von 1 : 10 bis 10 : 1 aufweist.

11. Zusammensetzung nach einem der vorangehenden Ansprüche, **dadurch gekennzeichnet,**
**dass** die Zusammensetzung einen Alkoholgehalt, insbesondere einen Ethanolgehalt, von weniger als 1,0 Vol.-%, vorzugsweise weniger als 0,85 Vol.-%, besonders bevorzugt weniger als 0,5 Vol.-%, ganz besonders bevorzugt weniger als 0,25 Vol.-%, noch mehr bevorzugt weniger als 0,1 Vol.-%, am meisten bevorzugt von etwa 0 Vol.-%, aufweist; und/oder
**dass** die Zusammensetzung unter Normalbedingungen, insbesondere bei einer Temperatur von 25 °C und unter einem Druck von 1,01325 bar, flüssig und/oder fließfähig ist; und/oder
**dass** der Pflanzenextrakt ein Droge/Extrakt-Verhältnis im Bereich von 5 : 1 bis 1 : 5, vorzugsweise im Bereich von 3 : 1 bis 1 : 4, bevorzugt im Bereich von 2 : 1 bis 1 : 3,5, ganz besonders bevorzugt im Bereich von 1 : 1 bis 1 : 3, aufweist; und/oder
**dass** die Zusammensetzung den mindestens einen Pflanzenextrakt, berechnet als Trockenextrakt und bezogen auf die Zusammensetzung, in Mengen von 0,001 bis 50 Gew.-%, insbesondere 0,01 bis 30 Gew.-%, vorzugsweise 0,05 bis 20 Gew.-%, besonders bevorzugt 0,1 bis 10 Gew.-%, enthält; und/oder
**dass** die Zusammensetzung wässrig basiert ist und/oder als wässrige Zubereitung formuliert ist und/oder einen wässrigen Exzipienten umfasst; und/oder
**dass** die Zusammensetzung Wasser enthält, insbesondere in Mengen von 5 bis 99 Gew.-%, insbesondere 7,5 bis 70 Gew.-%, vorzugsweise 10 bis 50 Gew.-%, besonders bevorzugt 15 bis 40 Gew.-%, bezogen auf die Zusammensetzung.

12. Zusammensetzung nach einem der vorangehenden Ansprüche, **dadurch gekennzeichnet,**
**dass** die Zusammensetzung mindestens einen vorzugsweise flüssigen Zucker und/oder Zuckeraustauschstoff, bevorzugt auf Basis einer Mischung von Saccharose und einem Sirup, insbesondere einem Sirup mit Glucose und Fructose, vorzugsweise in etwa gleichem Stoffmengenanteil (Invert-Sirup), enthält, insbesondere wobei die Zusammensetzung den mindestens einen Zucker und/oder Zuckeraustauschstoff in Mengen von 5 bis 95 Gew.-%, insbesondere 10 bis 90 Gew.-%, vorzugsweise 20 bis 90 Gew.-%, besonders bevorzugt 40 bis 85 Gew.-%, ganz besonders bevorzugt 60 bis 85 Gew.-%, enthält, bezogen auf die Zusammensetzung; und/oder
**dass** die Zusammensetzung mindestens ein Additiv, aus der Gruppe von Konservierungsmitteln, Stabilisatoren, geschmacks- und/oder geruchsmodifizierenden Substanzen, Rheologiemodifikatoren, Verdickungsmitteln, Farbstoffen, Puffern, pH-Stellmitteln, Emulgatoren, Suspensionsmitteln oder deren Mischungen, enthält, insbesondere wobei die Zusammensetzung das mindestens eine Additiv in Mengen von 0,001 bis 30 Gew.-%, insbesondere 0,01 bis 20 Gew.-%, vorzugsweise 0,05 bis 10 Gew.-%, besonders bevorzugt 0,1 bis 5 Gew.-%, enthält, bezogen auf die Zusammensetzung.

13. Zusammensetzung, insbesondere pharmazeutische Zubereitung, vorzugsweise zur prophylaktischen und/oder therapeutischen Behandlung von Atemwegserkrankungen, Erkältungskrankheiten sowie grippalen Infekten und damit verbundenen Symptomen, wie in einem der vorangehenden Ansprüche definiert, wobei die Zusammensetzung in Form einer zumindest im Wesentlichen alkoholfreien Zubereitung vorliegt, wobei die Zusammensetzung einen Alkoholgehalt, insbesondere einen Ethanolgehalt, von weniger als 2 Vol.-% aufweist, und mindestens die folgenden Inhaltsstoffe enthält:
mindestens einen Pflanzenextrakt mit sekretolytischer und/oder expektorationsfördernder Wirkung, jeweils mit einem Droge/Extrakt-Verhältnis im Bereich von 1 : 10 bis 10 : 1, insbesondere im Bereich von 5 : 1 bis 1 : 5, vorzugsweise im Bereich von 3 : 1 bis 1 : 4, bevorzugt im Bereich von 2 : 1 bis 1 : 3,5, ganz besonders bevorzugt im Bereich von 1 : 1 bis 1 : 3, insbesondere wobei die Zusammensetzung den mindestens einen Pflanzenextrakt, berechnet als Trockenextrakt, in Mengen von 0,001 bis 50 Gew.-%, insbesondere 0,01 bis 30 Gew.-%, vorzugsweise 0,05 bis 20 Gew.-%, besonders bevorzugt 0,1 bis 10 Gew.-%, enthält und/oder insbesondere wobei der mindestens eine Pflanzenextrakt mit sekretolytischer und/oder expektorationsfördernder Wirkung ausgewählt ist aus der Gruppe von Thymian-Extrakt (*Thymus vulgaris*), Primelwurzel-Extrakt (*Primula*), Efeu-Extrakt (*Hedera helix*), Holunder-Extrakt (*Sambucus nigra*), Fenchel-Extrakt (*Foeniculum vulgare*), Huflattich-Extrakt (*Tussilago farga*), Anis-Extrakt (*Pimpinella anisa*) und Tüpfelfarn-Extrakt (*Polypodium vul*gare) sowie Mischungen und Kombinationen von mindestens zwei der vorgenannten Extrakte;
• Wasser, insbesondere in Mengen von 5 bis 99 Gew.-%, insbesondere 7,5 bis 70 Gew.-%, vorzugsweise 10 bis 50 Gew.-%, besonders bevorzugt 15 bis 40 Gew.-%;
• gegebenenfalls mindestens einen vorzugsweise flüssigen Zucker und/oder Zuckeraustauschstoff, bevorzugt auf Basis einer Mischung von Saccharose und einem Sirup, insbesondere einem Sirup mit Glucose und Fructose, vorzugsweise in etwa gleichem Stoffmengenanteil (Invert-Sirup), insbesondere wobei die Zusammensetzung den mindestens einen Zucker und/oder Zuckeraustauschstoff in Mengen von 5 bis 95 Gew.-%, insbesondere 10 bis 90 Gew.-%, vorzugsweise 20 bis 90 Gew.-%, besonders bevorzugt 40 bis 85 Gew.-%, ganz besonders bevorzugt 60 bis 85 Gew.-%, enthält;
• mindestens ein Additiv aus der Gruppe von Netzmitteln und/oder oberflächenaktiven Substanzen, insbesondere wobei die Zusammensetzung das mindestens eine Additiv in Mengen von 0,001 bis 30 Gew.-%, insbesondere 0,01 bis 20 Gew.-%, vorzugsweise 0,05 bis 10 Gew.-%, besonders bevorzugt 0,1 bis 5 Gew.-%, enthält;
wobei alle vorgenannten Gewichtsprozentangaben auf die Zusammensetzung bezogen sind.

14. Zusammensetzung, insbesondere pharmazeutische Zubereitung, nach einem der vorangehenden Ansprüche zur Verwendung bei der prophylaktischen und/oder therapeutischen Behandlung von Atemwegserkrankungen, Erkältungskrankheiten sowie grippalen Infekten und damit verbundenen Symptomen.

15. Verwendung einer Zusammensetzung nach einem der vorangehenden Ansprüche zur Herstellung eines Arzneimittels zur prophylaktischen und/oder therapeutischen Behandlung von Atemwegserkrankungen, Erkältungskrankheiten sowie grippalen Infekten und damit verbundenen Symptomen.

## Claims

1. Method for preparing an at least substantially alcohol-free composition on the basis of at least one plant extract with a secretion and/or expectoration promoting effect, wherein the composition has an alcohol content, particularly an ethanol content, of less than 2 vol.-%,
wherein the method comprises the following method stages:
(a) provision of at least one alcoholic, especially ethanolic, plant extract with a secretion and/or expectoration promoting effect with a drug/extract ratio in the range of 1:10 to 10:1, wherein the at least one plant extract with a secretion and/or expectoration promoting effect is selected from the group of thyme extract (*Thymus vulgaris*), primrose extract (*Primula*), ivy extract (*Hedera helix*), elderberry extract (*Sambucus nigra*), fennel extract (*Foeniculum vulgare*)*,* coltsfoot extract (*Tussilago farfara*), anise extract (*Pimpinella anisum*) and polypody extract (*Polypodium vulgare*), and mixtures and combinations of at least two of the aforementioned extracts; and
(b) subsequent removal of the alcohol, in particular ethanol, by means of membrane filtration in order to obtain a substantially alcohol-free composition with an alcohol, particularly an ethanol, content of less than 2 vol.-%, based on at least a plant extract with a secretion and/or expectoration promoting effect, wherein at least a dispersing or wetting agent, preferably a solubilizing agent, is added prior to performing the membrane filtration, and/or wherein the membrane filtration is performed in the presence of at least a dispersing or wetting agent, preferably a solubilizing agent, wherein the dispersing or wetting agent is selected from the group of organic oils, fats and fatty acids, lecithins, glycerides and combinations thereof, and wherein the dispersing or wetting agent is used in amounts from 0.1 to 30 wt.-%, based on the total mixture of alcoholic plant extract, additive, water and optionally other ingredients, to be subjected to membrane filtration.

2. Method according to claim 1, **characterized in that**
the at least one alcoholic plant extract provided in method stage (a) has a drug/extract ratio in the range from 5:1 to 1:5, preferably in the range from 3:1 to 1:4, more preferably in the range from 2:1 to 1:3.5, most preferably in the range from 1:1 to 1:3; and/or
the alcoholic plant extract provided in method stage (a) is an ethanol-based plant extract; and/or
the at least one alcoholic plant extract provided in method stage (a) has an alcohol, in particular ethanol, content of more than 10 vol.-% and/or more than 10 wt.-%, in particular more than 20 vol.-% and/or more than 20 wt.-%, particularly preferably more than 30 vol.-% and/or more than 30 wt.-%, and/or that the at least one alcoholic plant extract provided in method stage (a) has an alcohol, in particular ethanol, content in the range from 10 vol.-% to 99 vol.-% and/or in the range of 10 wt.-% to 99 wt.-%, in particular in the range of 20 vol.-% to 95 vol.-% and/or in the range of 20 wt .-% to 95 wt.-%, particularly preferably in the range of 30 vol.-% to 90 vol.-% and/or in the range of 30 wt.-% to 90 wt.-%.

3. Method according to any one of the preceding claims, **characterized in that**
the membrane filtration in method stage (b) is performed as ultrafiltration, nanofiltration, reverse osmosis (hyperfiltration) or dialysis, preferably as reverse osmosis (hyperfiltration) or dialysis, preferably as reverse osmosis (hyperfiltration); and/or
the membrane filtration, particularly reverse osmosis (hyperfiltration), is performed with a pressure difference and/or a pressure gradient and/or a hydrostatic pressure difference in the range of 10 to 100 bar, in particular 20 to 90 bar, preferably 30 to 80 bar.

4. Method according to any one of the preceding claims, **characterized in that** the membrane filtration in method stage (b) is carried out using at least one membrane filter, in particular wherein
• the membrane filter is made of inorganic or organic materials, especially organic materials, preferably polymeric organic materials, in particular wherein the membrane material is selected from the group of (i) fluorinated and perfluorinated organic polymers, in particular fluorinated and perfluorinated olefins, preferably polytetra-fluoroethylenes; (ii) materials based on cellulose or cellulose derivatives, especially cellulose acetates and regenerated cellulose; (iii) sulfonated organic polymers, in particular polysulphones and polyethersulphones; (iv) polyolefins, particularly polyethylenes and polypropylenes; (v) polyamides; (vi) polyesters and combinations thereof; and/or
• the membrane filter is formed with one or more layers, in particular as a composite; and/or
• the membrane filter is fiber reinforced; and/or
• the membrane filter has a pore size of not more than 0.001 µm, in particular in the range of 0.0001 to 0.001 µm; and/or
• the membrane filter has a selective permeability and/or porosity, in particular a selective permeability with respect to alcohol, especially ethanol, and/or that the membrane filter is porous, preferably selectively porous, with respect to alcohol, especially ethanol; and/or
• the membrane filter is impermeable with respect to water.

5. Method according to any one of the preceding claims, **characterized in that**
the membrane filtration in method stage (b) is carried out at temperatures in the range of 5 to 80°C, especially 10 to 70°C, preferably 15 to 60°C, particularly preferably 20 to 50°C; and/or
the membrane filtration in method stage (b) is carried out continuously or discontinuously, preferably continuously; and/or
the membrane filtration in method stage (b) is carried out in an open or closed system, preferably in a closed system; and/or
water is added to the alcohol, in particular ethanol, plant extract provided in stage (a) prior to performing the membrane filtration, in particular wherein the alcohol content of the plant extract is adjusted to a value of at most 40 vol.-% and/or 40 wt.-%, in particular at most 35 vol.-% and/or 35 wt.-%, preferably at most 30 vol.-% and/or 30 wt.-%; and/or
the at least one dispersing or wetting agents is used in quantities of 0.5 to 20 wt.-%, preferably 1 to 15 wt.-%, based on the total mixture of alcoholic plant extract additive, water and optionally other ingredients to be subjected to membrane filtration.

6. Method according to any one of the preceding claims, **characterized in that**
the membrane filtration in method stage (b) is carried out in one or more stages, preferably several stages; and/or
the membrane filtration in method stage (b) is carried out in stages, preferably in at least two stages, especially at least three consecutive stages (sub-stages), particularly preferably in three to ten successive stages (sub-stages), in particular wherein the membrane filtration is carried out at each stage in such a way that the alcohol content, in particular the ethanol content, is reduced at each stage by at least 20%, particularly by at least 30%, preferably at least 40%, based on the starting alcohol content at the beginning of each stage, and/or in particular wherein water is added to each composition at the beginning of each stage prior to performing the membrane filtration; and/or
the substantially alcohol-free composition resulting from method stage (b) is obtained as the retentate of the membrane filtration on the basis of at least one plant extract with a secretion and/or expectoration promoting effect.

7. Method according to any one of the preceding claims, **characterized in that**
• method stage (b) is followed by a stage (c), wherein the substantially alcohol-free composition resulting from stage (b) and based on at least one plant extract with secretion and/or expectoration promoting effect is adjusted to a desired final composition, especially in a form for oral administration;
• in particular, wherein water and/or an aqueous excipient and/or optionally at least one further ingredient is added in stage (c) to the substantially alcohol-free composition resulting from method stage (b) and based on at least one plant extract with a secretion and/or expectoration promoting effect;
• in particular wherein the aqueous excipient used in method stage (c) has, in addition to water, at least one preferably liquid sugar, sugar substitute and/or carbohydrate, preferably based on a mixture of sucrose and a syrup, in particular a syrup containing glucose and fructose, preferably in approximately the same amount of substance (invert syrup), and/or that the aqueous excipient used in method stage (c) has, in addition to water, at least one further additive, in particular from the group of dispersing or wetting agents and/or surfactants, preservatives, stabilizers, taste and/or smell modifying substances, rheology modifiers, thickeners, dyes, buffers, pH adjusting agents, emulsifying agents, suspension agents or mixtures thereof, and/or **in that** the aqueous excipient used in method stage (c) contains water in amounts from 5 to 95 wt.-%, especially 10 to 90 wt.-%, preferably 20 to 80 wt.-%, based on the excipient; and/or
• in particular wherein the further ingredient used in method stage (c) is selected from the group of, preferably, liquid sugars, sugar substitutes and/or carbohydrates, preferably based on a mixture of sucrose and a syrup, in particular a syrup containing glucose and fructose, preferably in about the same amount of substance (invert syrup), and/or additives, in particular wetting agents and/or surface-active substances, preservatives, stabilizers, taste and/or smell modifying substances, rheology modifiers, thickeners, dyes, buffers, pH adjusting agents, emulsifying agents, suspension agents or their mixtures.

8. Method according to any one of the preceding claims, **characterized in that**
the composition in stage (b) and/or (c) is adjusted to an alcohol content, more particularly to an ethanol content, of less than 1.0 vol.-%, preferably less than 0.85 Vol.-%, more preferably less than 0.5 vol.-%, most preferably less than 0.25 Vol.-%, particularly preferably less than 0.1 vol.-%, most particularly preferably to about 0 vol .-%; and/or
the composition is adjusted to a liquid and/or fluid viscosity or consistency under normal conditions, especially at a temperature of 25°C and under a pressure of 1.01325 bar.

9. Composition based on at least a plant extract with a secretion and/or expectoration promoting effect, in particular a pharmaceutical composition, preferably for use for prophylactic and/or therapeutic treatment of respiratory diseases, colds and influenza infections and associated symptoms, and that is obtainable by a method according to any one of the preceding claims.

10. Composition, in particular a pharmaceutical composition, preferably for use for prophylactic and/or therapeutic treatment of respiratory diseases, colds and influenza infections and associated symptoms, and that is obtainable by a method according to any one of the preceding claims, wherein the composition is in the form of an at least substantially alcohol-free preparation, wherein the composition has an alcohol content, particularly an ethanol content, of less than 2 vol.-%, wherein the composition comprises at least one dispersing or wetting agent, preferably a solubilizing agent, wherein the dispersing or wetting agent is selected from the group of organic oils, fats and fatty acids, lecithin, glycerides, and combinations thereof, and wherein the at least one plant extract with a secretion and/or expectoration promoting effect is selected from the group consisting of thyme extract *(Thymus vulgaris),* primrose extract (*Primula*), ivy extract (*Hedera helix*), elderberry extract (*Sambucus nigra*), fennel extract (*Foeniculum vulgare*), coltsfoot extract (*Tussilago farfara*), anise extract (*Pimpinella anisum*) and polypody extract (*Polypodium vulgare*)*,* and mixtures and combinations of at least two of the aforementioned extracts, wherein the plant extract has a drug/extract ratio in the range of 1:10 to 10:1

11. Composition according to any one of the preceding claims, **characterized in that**
the composition has an alcohol content, more particularly an ethanol content, of less than 1.0 vol.-%, preferably less than 0.85 vol.-%, more preferably less than 0.5 vol.-%, most preferably less than 0.25 vol.-%, particularly preferably less than 0.1 vol.-%, even more particularly preferably about 0 vol.-%; and/or
the composition is liquid and/or flowable under normal conditions, especially at a temperature of 25°C and under a pressure of 1.01325 bar; and/or
the plant extract has a drug/extract ratio in the range from 5:1 to 1:5, preferably in the range from 3:1 to 1:4, more preferably in the range from 2:1 to 1:3.5, most preferably in the range from 1:1 to 1:3; and/or
the composition contains the at least one plant extract, calculated as dry extract and based on the composition, in amounts of from 0.001 to 50 wt.%, in particular 0.01 to 30 wt.-%, preferably 0.05 to 20 wt.-%, particularly preferably 0.1 to 10 wt .-%; and/or
the composition is water based and/or is formulated as an aqueous preparation and/or comprises an aqueous excipient; and/or
the composition contains water, in particular in amounts of from 5 to 99 wt.-%, in particular from 7.5 to 70 wt.-%, preferably 10 to 50 wt.-%, particularly preferably 15 to 40 wt.-%, based on the composition.

12. Composition according to any one of the preceding claims, **characterized in that**
the composition contains at least a preferably liquid sugar and/or sugar substitute, preferably based on a mixture of sucrose and a syrup, in particular a syrup containing glucose and fructose, preferably in about the same substance fraction (invert syrup), in particular wherein the composition contains the at least one sugar and/or sugar substitute in amounts from 5 to 95 wt.-%, especially 10 to 90 wt.-%, preferably 20 to 90 wt.-%, particularly preferably 40 to 85 wt.-%, most preferably 60 to 85 wt.-%, based on the composition; and/or
the composition contains at least one additive from the group of preservatives, stabilizers, taste and/or smell modifying substances, rheology modifiers, thickeners, dyes, buffers, pH adjusting agents, emulsifying agents, suspension agents or mixtures thereof, in particular wherein the composition comprises at least an additive in amounts of 0.001 to 30 wt.%, in particular 0.01 to 20 wt.-%, preferably 0.05 to 10 wt.-%, particularly preferably 0.1 to 5 wt.-%, based on the composition.

13. Composition, in particular a pharmaceutical composition, preferably for the prophylactic and/or therapeutic treatment of respiratory diseases, colds and influenza infections and associated symptoms, as defined in any of the preceding claims, wherein the composition is in the form of an at least substantially alcohol-free preparation, wherein the composition has an alcohol content, particularly an ethanol content, of less than 2 vol.-%, and at least the following ingredients:
at least one plant extract with a secretion and/or expectoration promoting effect with a drug/extract ratio in the range from 1:10 to 10:1, in particular in the range from 5:1 to 1:5, preferably in the range from 3:1 to 1:4, more preferably in the range of 2:1 to 1:3.5, most preferably in the range from 1:1 to 1:3, in particular wherein the composition contains the at least one plant extract, calculated as dry extract, in amounts of 0.001 to 50 wt.-%, in particular 0.01 to 30 wt.-%, preferably 0.05 to 20 wt.-%, particularly preferably 0.1 to 10 wt.-%, and/or in particular wherein the at least one plant extract with a secretion and/or expectoration promoting effect is selected from the group of thyme extract (*Thymus vulgaris*)*,* primrose extract (*Primula*)*,* ivy extract *(Hedera helix),* elderberry extract (*Sambucus nigra*), fennel extract (*Foeniculum vulgare*), coltsfoot extract (*Tussilago farfara*), anise extract (*Pimpinella anisum*) and polypody extract (*Polypodium vulgare*), and mixtures and combinations of at least two of the aforementioned extracts;
• water, in particular in amounts of from 5 to 99 wt.-%, in particular from 7.5 to 70 wt.-%, preferably 10 to 50 wt.-%, particularly preferably from 15 to 40%.
• optionally at least a preferably liquid sugar and/or sugar substitute, preferably based on a mixture of sucrose and a syrup, in particular a syrup containing glucose and fructose, preferably in about the same substance fraction (invert syrup), in particular wherein the composition contains the at least one sugar and/or sugar substitute in amounts of from 5 to 95 wt.-%, especially 10 to 90 wt.-%, preferably 20 to 90 wt.-%, particularly preferably 40 to 85 wt.-%, most preferably from 60 to 85 wt.-%;
• at least one additive from the group of wetting agents and/or surface-active substances, in particular wherein the composition contains at least one additive in amounts of 0.001 to 30 wt.-%, in particular 0.01 to 20 wt.-%, preferably 0.05 to 10 wt.-%, particularly preferably 0.1 to 5 wt .-%;
wherein all the aforementioned weight percentages are based on the composition.

14. Composition, in particular a pharmaceutical composition according to any one of the preceding claims for use in the prophylactic and/or therapeutic treatment of respiratory diseases, colds and influenza infections and associated symptoms.

15. Use of a composition according to any one of the preceding claims for the manufacture of a medicament for the prophylactic and/or therapeutic treatment of respiratory diseases, colds and influenza infections and associated symptoms.

## Revendications

1. Procédé de fabrication d'une composition au moins pour l'essentiel sans alcool, à base d'au moins un extrait de plante ayant un effet sécrétolytique et/ou favorisant l'expectoration, la composition présentant une teneur en alcool, en particulier une teneur en méthanol, inférieure à 2 % en volume,
le procédé comprenant les étapes de procédé suivantes :
(a) mise à disposition d'au moins un extrait de plante alcoolique, en particulier éthanolique, ayant un effet sécrétolytique et/ou favorisant l'expectoration, présentant un rapport drogue/extrait compris dans la plage de 1:10 à 10:1, le ou les extraits de plantes ayant un effet sécrétolytique et/ou favorisant l'expectoration étant choisis dans le groupe consistant en l'extrait de thym (*Thymus vulgaris*), l'extrait de primevère (*Primula*), l'extrait de lierre (*Hedera helix*)*,* l'extrait de sureau (*Sambucus nigra*), l'extrait de fenouil (*Foeniculum vulgare*), l'extrait de tussilage (*Tussilago farfara*), l'extrait d'anis (*Pimpinella anisum*) et l'extrait de polypode commun (*Polypodium vulgare*), ainsi que les mélanges et les combinaisons d'au moins deux des extraits mentionnés ci-dessus ; et
(b) puis élimination de l'alcool, en particulier de l'éthanol, par une filtration sur membrane, pour obtenir une composition pour l'essentiel sans alcool, ayant une teneur en alcool, en particulier une teneur en éthanol, inférieure à 2 % en volume, à base d'au moins un extrait de plante ayant un effet sécrétolytique et/ou favorisant l'expectoration, au moins un dispersant ou un mouillant, de préférence un promoteur d'adhérence, étant ajouté avant la mise en oeuvre de la filtration sur membrane, et/ou la filtration sur membrane étant mise en oeuvre en présence d'au moins un dispersant ou d'un mouillant, de préférence d'un promoteur d'adhérence, le dispersant ou le mouillant étant choisi dans le groupe consistant en les huiles, graisses et acides gras organiques, les lécithines, les glycérides ainsi que leurs combinaisons, et les dispersants ou les mouillants étant utilisés en des quantités de 0,1 à 30 % en poids par rapport au mélange total devant subir la filtration sur membrane, constitué d'un extrait de plante alcoolique, d'un additif, d'eau et éventuellement d'autres constituants.

2. Procédé selon la revendication 1, **caractérisé en ce que** dans l'étape de procédé (a), le ou les extraits de plantes alcooliques sont mis à disposition avec un rapport drogue/extrait compris dans la plage de 5:1 à 1:5, de préférence dans la plage de 3:1 à 1:4, préférentiellement dans la plage de 2:1 à 1:3,5, d'une manière plus particulièrement préférée dans la plage de 1:1 à 1:3 ; et/ou
l'extrait de plante alcoolique mis à disposition dans l'étape de procédé (a) est un extrait de plante à base d'éthanol ; et/ou
dans l'étape de procédé (a), on met à disposition le ou les extraits de plantes alcooliques ayant une teneur en alcool, en particulier en éthanol, supérieure à 10 % en volume et/ou supérieure à 10 % en poids, en particulier supérieure à 20 % en volume et/ou supérieure à 20 % en poids, d'une manière particulièrement préférée supérieure à 30 % en volume et/ou supérieure à 30 % en poids, et/ou
dans l'étape de procédé (a), on met à disposition le ou les extraits de plantes alcooliques ayant une teneur en alcool, en particulier en éthanol, comprise dans la plage de 10 % en volume à 99 % en volume et/ou dans la plage de 10 % en poids à 99 % en poids, en particulier dans la plage de 20 % en volume à 95 % en volume et/ou dans la plage de 20 % en poids à 95 % en poids, d'une manière particulièrement préférée dans la plage de 30 % en volume à 90 % en volume et/ou dans la plage de 30 % en poids à 90 % en poids.

3. Procédé selon l'une des revendications précédentes, **caractérisé en ce que**
dans l'étape de procédé (b), on met en oeuvre la filtration sur membrane sous forme d'une ultrafiltration, d'une nanofiltration, d'une osmose inverse (hyperfiltration) ou d'une dialyse, de préférence sous forme d'une osmose inverse (hyperfiltration) ou d'une dialyse, préférentiellement sous forme d'une osmose inverse (hyperfiltration) ;
et/ou
on met en oeuvre la filtration sur membrane, en particulier l'osmose inverse (hyperfiltration), avec une différence de pression et/ou un gradient de pression et/ou une différence de pression hydrostatique, compris dans la plage de 10 à 100 bar, en particulier de 20 à 90 bar, de préférence de 30 à 80 bar.

4. Procédé selon l'une des revendications précédentes, **caractérisé en ce que**, dans l'étape de procédé (b), on met en oeuvre la filtration sur membrane à l'aide d'au moins une membrane filtrante, en particulier dans lequel
- la membrane filtrante est constituée de matériaux inorganiques ou organiques, en particulier de matériaux organiques, de préférence de matériaux organiques polymères, en particulier dans lequel le matériau de la membrane est choisi dans le groupe consistant en (i) les polymères organiques fluorés et perfluorés, en particulier les oléfines fluorées et perfuorées, de préférence les polytétrafluoréthylènes ; (ii) les matériaux à base de cellulose ou de dérivés de cellulose, en particulier les acétates de cellulose et la cellulose régénérée ; (iii) les polymères organiques sulfonés, en particulier les polysulfones et les polyéthersulfones ; (iv) les polyoléfines, en particulier les polyéthylènes et les polypropylènes ; (v) les polyamides ; (vi) les polyesters, ainsi que leurs combinaisons, et/ou
- la membrane filtrante est conçue avec une structure monocouche ou multicouche, en particulier sous forme d'un matériau composite ; et/ou
- la membrane filtrante est conçue renforcée par des fibres ; et/ou
- la membrane filtrante présente une grosseur de pores d'au plus 0,001 µm, en particulier comprise dans la plage de 0,0001 à 0,001 µm ; et/ou
- la membrane filtrante présente une perméabilité et/ou une pénétrabilité sélectives, en particulier est conçue de façon à permettre une pénétration sélective de l'alcool, en particulier l'éthanol, et/ou que la membrane filtrante présente une pénétrabilité, de préférence une pénétrabilité sélective vis-à-vis de l'alcool, en particulier l'éthanol ; et/ou
- la membrane filtrante est conçue imperméable à l'eau.

5. Procédé selon l'une des revendications précédentes, **caractérisé en ce que**
dans l'étape de procédé (b), la filtration sur membrane est mise en oeuvre à des températures comprises dans la plage de 5 à 80°C, en particulier de 10 à 70°C, de préférence de 15 à 60°C, d'une manière particulièrement préférée de 20 à 50°C ; et/ou
dans l'étape de procédé (b), la filtration sur membrane est mise en oeuvre en continu ou d'une manière discontinue, de préférence en continu ; et/ou
dans l'étape de procédé (b), la filtration sur membrane est mise en oeuvre dans un système ouvert ou fermé, de préférence dans un système fermé ; et/ou
on ajoute de l'eau, avant mise en oeuvre de la filtration sur membrane, à l'extrait de plante alcoolique, en particulier éthanolique, mis à disposition dans l'étape de procédé (a), en particulier la proportion de l'alcool dans l'extrait de plante étant ajustée à une valeur d'au plus 40 % en volume et/ou 40 % en poids, en particulier d'au plus 35 % en volume et/ou 35 % en poids, de préférence d'au plus 30 % en volume et/ou 30 % en poids ; et/ou
on utilise le ou les dispersants ou mouillants en des quantités de 0,5 à 20 % en poids, de préférence de 1 à 15 % en poids, par rapport au mélange complet destiné à être soumis à la filtration sur membrane, constitué de l'extrait de plante alcoolique, d'un additif, d'eau et éventuellement d'autres constituants.

6. Procédé selon l'une des revendications précédentes, **caractérisé en ce que**
dans l'étape de procédé (b), la filtration sur membrane est mise en oeuvre en une ou plusieurs étapes, de préférence en plusieurs étapes ; et/ou
dans l'étape de procédé (b), la filtration sur membrane est mise en oeuvre en plusieurs étapes, de préférence en au moins deux, en particulier en au moins trois étapes successives (étapes partielles), d'une manière particulièrement préférée en trois à dix étapes successives (étapes partielles), en particulier la filtration sur membrane étant dans chaque étape mise en oeuvre de telle sorte que la teneur en alcool, en particulier la teneur en éthanol subisse une réduction à chaque étape d'au moins 20 %, en particulier d'au moins 30 %, de préférence d'au moins 40 %, par rapport à la teneur initiale en alcool au début de l'étape considérée, et/ou de l'eau étant ajoutée au début de chaque étape à la composition considérée, avant la mise en oeuvre de la filtration sur membrane ; et/ou
on obtient sous forme d'un rétentat de la filtration sur membrane la composition obtenue dans l'étape de procédé (b), pour l'essentiel sans alcool, à base d'au moins un extrait de plante, ayant un effet sécrétolytique et/ou favorisant l'expectoration.

7. Procédé selon l'une des revendications précédentes, **caractérisé en ce que** l'étape de procédé (b) est suivie d'une étape de procédé (c), dans laquelle la composition obtenue dans l'étape de procédé (b), pour l'essentiel sans alcool, à base d'au moins un extrait de plante, ayant un effet sécrétolytique et/ou favorisant l'expectoration, est ajustée à une composition finale souhaitée, en particulier sous une forme adaptée à une administration par voie orale ;
- en particulier dans lequel, dans l'étape de procédé (c), on ajoute de l'eau et/ou un excipient aqueux et/ou éventuellement au moins un constituant supplémentaire à la composition obtenue dans l'étape de procédé (b), pour l'essentiel sans alcool, à base d'au moins un extrait de plante, ayant un effet sécrétolytique et/ou favorisant l'expectoration ;
- en particulier dans lequel l'excipient aqueux utilisé dans l'étape de procédé (c) contient, outre de l'eau, au moins un sucre, un succédané de sucre et/ou un glucide de préférence liquide, de préférence à base d'un mélange de saccharose et d'un sirop, en particulier d'un sirop contenant du glucose et du fructose, de préférence selon des proportions pondérales approximativement identiques (sirop inverti), et/ou que l'excipient utilisé dans l'étape de procédé (c) contient, outre de l'eau, au moins un additif supplémentaire, en particulier du groupe consistant en les dispersants ou mouillants et/ou les substances tensioactives, les agents de conservation, les stabilisants, les substances modifiant le goût et/ou l'odeur, les agents modifiant la rhéologie, les épaississants, les colorants, les tampons, les agents d'ajustement du pH, les émulsifiants, les agents de mise en suspension ou les mélanges de ceux-ci, et/ou que l'excipient aqueux utilisé dans l'étape de procédé (c) contient de l'eau en des quantités de 5 à 95 % en poids, en particulier de 10 à 90 % en poids, de préférence de 20 à 80 % en poids, par rapport à l'excipient ; et/ou
- en particulier dans lequel le constituant supplémentaire utilisé dans l'étape de procédé (c) est choisi dans le groupe consistant en les sucres, succédanés du sucre et/ou glucides de préférence liquides, de préférence à base d'un mélange de saccharose et d'un sirop, en particulier d'un sirop contenant du glucose et du fructose, de préférence selon des proportions pondérales approximativement égales (sirop inverti), et/ou les additifs, en particulier les mouillants et/ou les substances tensioactives, les agents de conservation, les stabilisants, les substances modifiant le goût et/ou l'odeur, les agents modifiant la rhéologie, les épaississants, les colorants, les tampons, les agents d'ajustement du pH, les émulsifiants, les agents de mise en suspension ou les mélanges de ceux-ci.

8. Procédé selon l'une des revendications précédentes, **caractérisé en ce que**
la composition est, dans l'étape de procédé (b) et/ou (c), ajustée à une teneur en alcool, en particulier à une teneur en éthanol, inférieure à 1,0 % en volume, de préférence inférieure à 0,85 % en volume, d'une manière particulièrement préférée inférieure à 0,5 % en volume, d'une manière tout particulièrement préférée inférieure à 0,25 % en volume, d'une manière encore plus préférée inférieure à 0,1 % en volume, d'une manière tout spécialement préférée à environ 0 % en volume ; et/ou
la composition est ajustée à une viscosité et une consistance liquide et/ou fluide dans les conditions normales, en particulier à une température de 25°C et sous une pression de 1,01325 bar.

9. Composition à base d'au moins un extrait de plante ayant un effet sécrétolytique et/ou favorisant l'expectoration, en particulier composition pharmaceutique, de préférence pour une utilisation pour le traitement prophylactique et/ou thérapeutique des maladies des voies respiratoires, des maladies du refroidissement ainsi que des infections grippales et des symptômes qui y sont associés, pouvant être obtenue par un procédé selon l'une des revendications précédentes.

10. Composition, en particulier préparation pharmaceutique, de préférence pour une utilisation pour le traitement prophylactique et/ou thérapeutique des maladies des voies respiratoires, des maladies du refroidissement ainsi que des infections grippales et des symptômes qui y sont associés, pouvant être obtenue par un procédé selon l'une des revendications précédentes, la composition se présentant sous forme d'une préparation au moins pour l'essentiel sans alcool, la composition présentant une teneur en alcool, en particulier une teneur en éthanol, inférieure à 2 % en volume, la composition contenant au moins un dispersant ou un mouillant, de préférence un promoteur d'adhérence, les dispersants ou les mouillants étant choisis dans le groupe consistant en les huiles, les graisses et les acides gras organiques, les lécithines, les glycérides ainsi que leurs combinaisons, et le ou les extraits de plantes ayant un effet sécrétolytique et/ou favorisant l'expectoration étant choisis dans le groupe consistant en l'extrait de thym (*Thymus vulgaris*), l'extrait de primevère (*Primula*), l'extrait de lierre (*Hedera helix*), l'extrait de sureau (*Sambucus nigra*), l'extrait de fenouil (*Foeniculum vulgare*), l'extrait de tussilage (*Tussilago farfara*), l'extrait d'anis (*Pimpinella anisum*) et l'extrait de polypode commun (*Polypodium vulgare*), ainsi que les mélanges et combinaisons d'au moins deux des extraits mentionnés ci-dessus, l'extrait de plante présentant un rapport drogue/extrait compris dans la plage de 1:10 à 10:1.

11. Composition selon l'une des revendications précédentes, **caractérisée en ce que**
la composition présente une teneur en alcool, en particulier une teneur en éthanol, inférieure à 1,0 % en volume, de préférence inférieure à 0,85 % en volume, d'une manière particulièrement préférée inférieure à 0,5 % en volume, d'une manière tout particulièrement préférée inférieure à 0,25 % en volume, d'une manière encore plus préférée inférieure à 0,1 % en volume, d'une manière tout spécialement préférée d'environ 0 % en volume ; et/ou
la composition est liquide et/ou fluide dans les conditions normales, en particulier à une température de 25°C et sous une pression de 1,01325 bar ; et/ou
l'extrait de plante présente un rapport drogue/extrait compris dans la plage de 5:1 à 1:5, de préférence dans la plage de 3:1 à 1:4, préférentiellement dans la plage de 2:1 à 1:3,5, d'une manière tout particulièrement préférée dans la plage de 1:1 à 1:3 ; et/ou
la composition contient le ou les extraits de plantes en une quantité, exprimée en extrait sec et rapportée à la composition, comprise entre 0,001 et 50 % en poids, en particulier entre 0,01 et 30 % en poids, de préférence entre 0,05 et 20 % en poids, d'une manière particulièrement préférée entre 0,1 et 10 % en poids ; et/ou
la composition est à base aqueuse et/ou est formulée sous forme d'une préparation aqueuse et/ou comprend un excipient aqueux ; et/ou
la composition contient de l'eau, en particulier en des quantités de 5 à 99 % en poids, en particulier de 7,5 à 70 % en poids, de préférence de 10 à 50 % en poids, d'une manière particulièrement préférée de 15 à 40 % en poids, par rapport à la composition.

12. Composition selon l'une des revendications précédentes, **caractérisée en ce que**
la composition contient au moins un sucre et/ou un succédané du sucre, de préférence liquide, de préférence à base d'un mélange de saccharose et d'un sirop, en particulier d'un sirop contenant du glucose et du fructose, de préférence selon des proportions pondérales approximativement égales (sirop inverti), la composition contenant le ou les sucres et/ou succédanés du sucre en des quantités de 5 à 95 % en poids, en particulier de 10 à 90 % en poids, de préférence de 20 à 90 % en poids, d'une manière particulièrement préférée de 40 à 85 % en poids, d'une manière plus particulièrement préférée de 60 à 85 % en poids, par rapport à la composition ; et/ou
la composition contient au moins un additif du groupe consistant en les agents de conservation, les stabilisants, les substances modifiant le goût et/ou l'odeur, les agents modifiant la rhéologie, les épaississants, les colorants, les tampons, les agents d'ajustement du pH, les émulsifiants, les agents de mise en suspension ou leurs mélanges, en particulier la composition contenant le ou les additifs en des quantités de 0,001 à 30 % en poids, en particulier de 0,01 à 20 % en poids, de préférence de 0,05 à 10 % en poids, d'une manière particulièrement préférée de 0,1 à 5 % en poids, par rapport à la composition.

13. Composition, en particulier préparation pharmaceutique, de préférence pour le traitement prophylactique et/ou thérapeutique des maladies des voies respiratoires, des maladies du refroidissement, ainsi que des infections grippales et des symptômes qui y sont associés, telle que définie dans l'une des revendications précédentes, la composition se présentant sous forme d'au moins une préparation pour l'essentiel sans alcool, la composition présentant une teneur en alcool, en particulier une teneur en méthanol, inférieure à 2 % en volume, et contient au moins les constituants suivantes :
au moins un extrait de plante ayant un effet sécrétolytique et/ou favorisant l'expectoration, chacun ayant un rapport drogue/extrait compris dans la plage de 1:10 à 10:1, en particulier dans la plage de 5:1 à 1:5, de préférence dans la plage de 3:1 à 1:4, préférentiellement dans la plage de 2:1 à 1:3,5, d'une manière tout particulièrement préférée dans la plage de 1:1 à 1:3, en particulier la composition contenant le ou les extraits de plantes en des quantités, exprimées en extrait sec, de 0,001 à 50 % en poids, en particulier de 0,01 à 30 % en poids, de préférence de 0,05 à 20 % en poids, d'une manière particulièrement préférée de 0,1 à 10 % en poids, et/ou en particulier le ou les extraits de plantes ayant un effet sécrétolytique et/ou favorisant l'expectoration sont choisis dans le groupe consistant en l'extrait de thym (*Thymus vulgaris*), l'extrait de primevère (*Primula*), l'extrait de lierre (*Hedera helix*), l'extrait de sureau (*Sambucus nigra*)*,* l'extrait de fenouil (*Foeniculum vulgare*), l'extrait de tussilage (*tussilage farfara*), l'extrait d'anis (*Pimpinella anisum*) et l'extrait de polypode commun (*Polypodium vulgare*), ainsi que les mélanges et combinaisons d'au moins deux des extraits mentionnés ;
- de l'eau, en particulier en des quantités de 5 à 99 % en poids, en particulier de 7,5 à 70 % en poids, de préférence de 10 à 50 % en poids, d'une manière particulièrement préférée de 15 à 40 % en poids ;
- éventuellement, au moins un sucre et/ou un succédané du sucre de préférence liquide, de préférence à base d'un mélange de saccharose et d'un sirop, en particulier d'un sirop contenant du glucose et du fructose, de préférence selon des proportions pondérales approximativement égales (sirop inverti), en particulier la composition contenant le ou les sucres et/ou succédanés du sucre en des quantités de 5 à 95 % en poids, en particulier de 10 à 90 % en poids, de préférence de 20 à 90 % en poids, d'une manière particulièrement préférée de 40 à 85 % en poids, d'une manière tout particulièrement préférée de 60 à 85 % en poids ;
- au moins un additif du groupe consistant en les mouillants et/ou les substances tensioactives, la composition contenant en particulier le ou les additifs en des quantités de 0,001 à 30 % en poids, en particulier de 0,01 à 20 % en poids, de préférence de 0,05 à 10 % en poids, d'une manière particulièrement préférée de 0,1 à 5 % en poids ;
toutes les indications de pourcentage en poids mentionnées ci-dessus étant rapportées à la composition.

14. Composition, en particulier préparation pharmaceutique, selon l'une des revendications précédentes, pour une utilisation lors du traitement prophylactique et/ou thérapeutique des maladies des voies respiratoires, des maladies du refroidissement ainsi que des infections grippales et des symptômes qui y sont associés.

15. Utilisation d'une composition selon l'une des revendications précédentes pour fabriquer un médicament destiné au traitement prophylactique et/ou thérapeutique des maladies des voies respiratoires, des maladies du refroidissement, ainsi que des infections grippales et des symptômes qui y sont associés.
